# EUROPEAN PATENT SPECIFICATION

(11) **EP 2 283 119 B1**
(45) Date of publication and mention of the grant of the patent: **07.01.2015**
(21) Application number: 09743599.4
(22) Date of filing: 06.05.2009
(51) Int. Cl.: C12N 5/071, C12N 5/07, C12N 5/02, A61K 35/12

(54) **METHODS AND COMPOSITIONS FOR INDUCING BROWN ADIPOGENESIS**
VERFAHREN UND ZUSAMMENSETZUNGEN ZUR EINLEITUNG VON BRAUNER ADIPOGENESE
PROCÉDÉS ET COMPOSITIONS POUR INDUIRE UNE ADIPOGENÈSE BRUNE

(30) Priority: 06.05.2008 US 50914
(43) Date of publication of application: 16.02.2011
(73) Proprietor: Joslin Diabetes Center, Inc., Boston, MA 02115 (US)
(72) Inventor: TSENG, Yu-hua, Newton, MA 02464 (US); SCHULZ, Tim Julius, Cambridge, MA 02139 (US)
(74) Representative: Fleck, Barbara
(86) International application number: PCT/US2009/043029
(87) International publication number: WO 2009/137613

(56) References cited:
- WO-A2-2005/037232
- WO-A2-2006/130690
- KR-A- 20070 085 288
- CASE JAMIE ET AL: "Clonal multilineage differentiation of murine common pluripotent stem cells isolated from skeletal muscle and adipose stromal cells", NEW YORK ACADEMY OF SCIENCES. ANNALS, WILEY-BLACKWELL PUBLISHING, INC, US, vol. 1044, 1 January 2005 (2005-01-01), pages 183-200, XP009139203, ISSN: 0077-8923
- TSENG YU-HUA ET AL: "New role of bone morphogenetic protein 7 in brown adipogenesis and energy expenditure", NATURE: INTERNATIONAL WEEKLY JOURNAL OF SCIENCE, NATURE PUBLISHING GROUP, UNITED KINGDOM, vol. 454, no. 7207, 21 August 2008 (2008-08-21), pages 1000-1004, XP002528628, ISSN: 0028-0836, DOI: 10.1038/NATURE07221
- SCHULZ TIM J ET AL: "Identification of inducible brown adipocyte progenitors residing in skeletal muscle and white fat", PROCEEDINGS OF THE NATIONAL ACADEMY OF SCIENCES OF THE UNITED STATES OF AMERICA, vol. 108, no. 1, January 2011 (2011-01), pages 143-148, XP002675155, ISSN: 0027-8424
- BARBARA CANNON ET AL.: 'Brown adipose tissue: function and physiological significance' PHYSIOL. REV. vol. 84, 2004, pages 277 - 359, XP008146196
- XIAOHUI JI ET AL.: 'Patterns of gene expression associated with BMP-2-induced osteoblast and adipocyte differentiation of mesenchymal progenitor cell 3T3-F442A' J. BONE MINER. METAB. vol. 18, 2000, pages 132 - 139, XP008146197
- EVAN D. ROSEN ET AL.: 'Adipocyte differentiation from the inside out' MOLECULAR CELL BIOLOGY vol. 7, 2006, pages 885 - 896, XP008146200

## Description

### FEDERALLY SPONSORED RESEARCH OR DEVELOPMENT

The U.S. Government has certain rights in this invention pursuant to Grant No. R01 DK077097, awarded by the National Institutes of Health.

### TECHNICAL FIELD

This disclosure relates to methods and compositions for treating obesity and weight-related diseases and disorders. More specifically, the disclosure provides methods and compositions for modulating (e.g., increasing) brown adipose tissue adipogenesis in a cell or population of cells, and methods for identifying cells capable of undergoing differentiation to or towards a brown adipose tissue cell lineage.

### BACKGROUND

Obesity is a major global health concern both directly and indirectly through its role in the development and pathogenesis of other disorders such as diabetes, heart disease, and cancer. Obesity develops when energy intake exceeds energy expenditure and is generally associated with an abnormal accumulation of fat cells or adipose tissue.

Adipose tissue, like muscle and bone, is of mesodermal origin, and contains a mitotic compartment that allows for growth and differentiation of white fat cells, also known as white adipose tissue (WAT) cells, and/or brown fat cells, also known as brown adipose tissue (BAT) cells. Both BAT and WAT cells arise from mesenchymal stem cells, which when triggered by the appropriate developmental cues become committed to the adipocyte lineage and are designated as preadipocytes. Preadipoxytes contain both WAT and BAT precursor or cells progenitor cells capable of differentiating into BAT or WAT cells.

Recently, a myogenic gene expression signature was identified in brown, but not white, fat precursor cells (Timmons et al., Proc. Natl. Acad. Sci. U.S.A., 104:4401-4406, 2007), which suggests that BAT precursor cells are distinct and distinguishable from WAT precursor cells. In addition, depots of brown adipocytes were found interspersed between hindlimb muscle bundles in mice, particularly in obesity-resistant strains (Almind et al., Proc. Natl. Acad. Sci. U.S.A., 104:2366-2371, 2007), suggesting that skeletal muscle may contain progenitor cells capable of differentiating to or towards BAT cells.

WAT tissue is the primary site of depot of triglycerides and release of fatty acids. WAT is found underneath the skin and provides heat insulation, cushioning against shock and jarring, and energy reserves. An average lean person has roughly 20 to 40 billion WAT cells. An obese person can have up to ten times more WAT than the average lean person.

BAT cells contain abundant and large mitochondria (Nedergaard et al., in Brown Adipose Tissue, Trayhurn and Nicholls, Eds. (Edward Arnold, Baltimore, 1986)), which serve as the center site for oxidative phosphorylation, intermediary metabolism, adaptive thermogenesis, generation of reactive oxygen species, and apoptosis. In BAT, mitochondrial biogenesis has been long known to accompany brown adipocyte differentiation. During the past decade, it has become increasingly evident that altered in mitochondrial integrity can contribute to a variety of human diseases, including obesity, diabetes, cancer, neurodegeneration, and aging (Duchen, Diabetes 53 (Suppl 1):S96-102 (2004); Taylor and Turnbull, Nat. Rev. Genet. 6:389-402 (2005); Lowell and Shulman, Science 307:384-387 (2005)).

BAT is involved in thermogenic energy expenditure through the expression of uncoupling protein-1 (UCP-1). BAT-dependent energy expenditure serves to maintain body temperature in the cold and/or waste food energy. Consequently, defective or insufficient BAT is frequently associated with obesity. The importance of BAT in overall energy homeostasis is underscored by the observation that BAT ablation in mice results in severe obesity, which is frequently accompanied by insulin resistance, hyperglycemia, hyperlipidemia, and hypercholesterolemia (Lowell at al., Nature 366(6457):740-2 (1993); Hamann et al., Diabetes. 44(11):1266-73 (1995); Hamann et al., Endocrinology 137(1):21-9 (1996).

### SUMMARY OF THE INVENTION

This disclosure is based, at least in part, on the discovery that stem cell antigen-1 positive (Sca-1+) progenitor cells treated with one or more bone morphogenic proteins (BMP) differentiate to or towards *bona fide* BAT cells. These BAT cells are genuine BAT cells with a complete capacity to respond to catecholamine stimulation by turning on the BAT cell thermogenic program. The invention relates to a method of increasing the number of cells with the characteristics of brown adipose tissue (BAT) cells in a population of cells *in vitro,* the method comprising:
contacting a population of cells comprising stem cell antigen-1 positive (Sca-1+) progenitor cells, wherein the population of progenitor cells comprises at least 60% Sca-1+ progenitor cells, *in* vitro with an effective amount of bone morphogenic protein (BMP)-7 and, optionally, up to three of bone morphogenic protein (BMP) -2, -4, or -6 or BMP-4 and, optionally, up to three of BMP-2, -6 or - 7 for a time sufficient to increase the number of cells with the characteristics of brown adipose tissue (BAT) cells in the population of cells; or
genetically engineering the population of cells to express bone morphogenic protein (BMP)-7 and, optionally, up to three of bone morphogenic protein (BMP) -2, -4, or -6 or BMP-4 and, optionally, up to three of BMP-2, -6 or -7 at a level sufficient to increase the number of cells with the characteristics of BAT cells in the population of cells.

As used herein, "BMP-treated" means that the cell has an artificially enhanced level of BMP signaling, e.g., BMP-2, -4, -5, -6, and/or -7 signaling. "Artificially" enhanced means that the level of BMP signaling has been increased by direct human intervention. BMP signaling can be enhanced by any method described herein, e.g., by treating the cell with a compound that enhances BMP signaling as described herein, e.g., BMP polypeptides, BMP encoding nucleic acids, small molecules, and/or antibodies. Populations of Sca-1+ cells activated by methods described herein are also included within the present invention. The cells can be autologous, allogeneic, or xenogeneic.

Methods described herein can include treating (e.g., contacting) a population of Sca-1+ progenitor cells with a compound in an amount sufficient to increase BMP signaling, thereby producing a population of BMP-treated cells.

Methods described herein can include implanting a population of these BMP-treated Sca-1+ cells into a subject. The BMP-treated Sca-1+ cells can be implanted directly or can be administered in a scaffold, matrix, or other implantable device to which the cells can attach (examples include carriers made of, e.g., collagen, fibronectin, elastin, cellulose acetate, cellulose nitrate, polysaccharide, fibrin, gelatin, and combinations thereof). In general, the methods described include implanting a population of BMP-treated Sca-1+ cells comprising a sufficient number of cells to promote brown adipogenesis in the subject, e.g., to increase the amount of BAT in the subject by at least 1%, e.g., 2%, 5%, 7%, 10%, 15%, 20%, 25% or more.

Methods described include providing a purified population of Sca-1+ progenitor cells, (e.g., a population of cells in which at least 60%, e.g., 70%, 80%, 90% or more of the cells are Sca-1+ progenitor cells); and contacting these cells with a compound capable of increasing the expression, levels or activity of one or more of BMP-2, -4, -5, -6, and/or -7, as described herein, thereby generating BMP-treated cells.
In the methods described herein, a compound that increases BMP-2, -4, -5, -6, and/or -7 signaling can be, e.g., one or more of the following:
(a) a BMP-2, -4, -5, -6, and/or -7 polypeptide or a functional fragment or variant thereof, preferably an active (e.g., BMPR-I and/or BMPR-II activating) BMP-2, -4, -5, -6, and/or -7 polypeptide or a functional fragment or analog thereof (e.g., a mature BMP-2, -4, -5, -6, and/or -7 polypeptide, e.g., a mature BMP-2, -4, -5, -6, and/or -7 polypeptide described herein);
(b) a peptide or protein agonist of BMP-2, -4, -5, -6, and/or -7 that increases the activity, e.g., the BMPR-I and/or BMPR-II activating activity of BMP-2, -4, -5, - 6, and/or -7 (e.g., by increasing or stabilizing binding of BMP-2, -4, -5, -6, and/or -7 to its receptor);
(c) a small molecule or protein mimetic that mimics BMP-2, -4, -5, -6, and/or - 7 signaling activity, e.g., BMPR-I and/or BMPR-II binding activity, or SMAD phosphorylating activity;
(d) a small molecule that increases expression of BMP-2, -4, -5, -6, and/or -7, e.g., by binding to the promoter region of a BMP-2, -4, -5, -6, and/or -7 gene;
(e) an antibody, e.g., an antibody that binds to and stabilizes or assists the binding of BMP-2, -4, -5, -6, and/or -7 to a BMP-2, -4, -5, -6, and/or -7 binding partner (e.g., a BMP-2, -4, -5, -6, and/or -7 receptor described herein). In some embodiments, the antibody that binds to the BMP-2, -4, -5, -6, and/or -7 is a monoclonal antibody, e.g., a humanized chimeric or human monoclonal antibody; or
(f) a nucleic acid encoding a BMP-2, -4, -5, -6, and/or -7 polypeptide or functional fragment or analog thereof. The nucleic acid can be a genomic sequence or a cDNA sequence.

As used herein, a "BMP-2, -4, -5, -6, and/or -7 polypeptide or nucleic acid" is a BMP-2, -4, -5, -6, and/or -7 polypeptide or nucleic acid as described herein, e.g., a mature human BMP-2, -4, -5, -6, and/or -7 polypeptide or active fragment thereof, or a nucleic acid encoding a mature human BMP-2, -4, -5, -6, and/or-7 polypeptide or active fragment thereof.

In some embodiments of the methods described herein, the compound is a BMP-2 polypeptide, e.g., human BMP-2, e.g., a mature BMP-2 polypeptide, e.g., a BMP-2 polypeptide that includes amino acids 283-396 of SEQ ID NO:1. The polypeptide can be a recombinant polypeptide.

In some embodiments of the methods described herein, the compound is a BMP-4 polypeptide, e.g., human BMP-4, e.g., a mature BMP-4 polypeptide, e.g., a BMP-4 polypeptide that includes amino acids 293-408 of SEQ ID NO:2. The polypeptide can be a recombinant polypeptide.

In some embodiments of the methods described herein, the compound is a BMP-5 polypeptide, e.g., human BMP-5, e.g., a mature BMP-5 polypeptide, e.g., a BMP-4 polypeptide that includes amino acids 323-454 of SEQ ID NO:3. The polypeptide can be a recombinant polypeptide.

In some embodiments of the methods described herein, the compound is a BMP-6 polypeptide, e.g., human BMP-6, e.g., a mature BMP-6 polypeptide, e.g., a BMP-6 polypeptide that includes amino acids 374-513 of SEQ ID NO:4, amino acids 382-513 of SEQ ID NO:4, amino acids 388-513 of SEQ ID NO:4, or amino acids 412-513 of SEQ ID NO:4. The polypeptide can be a recombinant polypeptide.

In some embodiments, the compound is a BMP-7 polypeptide, e.g., human BMP-7, e.g., a mature BMP-7 polypeptide, e.g., a BMP-7 polypeptide that includes amino acids 293-431 of SEQ ID NO:5. The polypeptide can be a recombinant polypeptide.

We also describe a nucleic acid encoding a BMP-2, -4, -5, -6, and/or -7 polypeptide, or a biologically active fragment or analog thereof. A BMP nucleic acid can include: a BMP-2, -4, -5, -6, and/or -7 coding region; a promoter sequence, e.g., a promoter sequence from a BMP-2, -4, -5, -6, and/or -7 gene or from another gene; an enhancer sequence; untranslated regulatory sequences, e.g., a 5' untranslated region (UTR), e.g., a 5'UTR from a BMP-2, -4, -5, -6, and/or -7 gene or from another gene, a 3' UTR, e.g., a 3'UTR from a BMP-2, -4, -5, -6, and/or -7 gene or from another gene; a polyadenylation site; an insulator sequence. We also describe that, the level of BMP-2, -4, -5, -6, and/or -7 protein is increased by increasing the level of expression of an endogenous BMP-2, -4, -5, -6, and/or -7 gene, e.g., by increasing transcription of the BMP-2, -4, -5, -6, and/or -7 gene or increasing BMP-2, -4, - 5, -6, and/or -7 mRNA stability. For example, transcription of the BMP-2, -4, -5, -6, and/or - 7 gene is increased by: altering the regulatory sequence of the endogenous BMP-2, -4, -5, -6, and/or -7 gene, e.g., by the addition of a positive regulatory element (such as an enhancer or a DNA-binding site for a transcriptional activator); the deletion of a negative regulatory element (such as a DNA-binding site for a transcriptional repressor) and/or replacement of the endogenous regulatory sequence, or elements therein, with that of another gene, thereby allowing the coding region of the BMP-2, -4, -5, -6, and/or -7 gene to be transcribed more efficiently.

For example, the nucleic acid encodes or increases transcription of BMP-7.

We also describe a population of BMP-treated Sca-1+ progenitor. For example, the cells are genetically engineered to express increased levels of a BMP-2, -4, -5, -6, and/or -7 polypeptide, e.g., a BMP-2, -4, -5, -6, and/or -7 polypeptide described herein, either stably or transiently. The cells can be, e.g., cultured mammalian cells, e.g., human cells. For example, the cells are genetically engineered to express at least one other protein as well, e.g., a non-BMP-2, -4, -5, -6, and/or -7 polypeptide, and/or a second (or more) BMP protein. The expressed BMP-2, -4, -5, -6, and/or -7 polypeptide will generally be of the same species as the stem cells, e.g., a human BMP expressed in human cells. For example, the cells are immortalized, e.g., capable of self-renewal indefinitely in culture.

We also describe methods of promoting brown adipogenesis in a subject. These methods include selecting a subject in need of treatment; obtaining a population of cells disclosed herein comprising stem cell antigen-1 positive (Sca-1+), CD45 negative, Mac 1 negative progenitor cells; and contacting the population of progenitor cells *in vitro* with an effective amount of a compound that promotes increased expression of one or more of bone morphogenic protein (BMP)-2, -4, -6, or -7 for a time sufficient to increase the number of cells with the characteristics of brown adipose tissue (BAT) cells in the population of cells; or genetically engineering the population of cells to express one or more of BMP-2, -4, -6, or -7 at a level sufficient to increase the number of cells with the characteristics of BAT cells in the population of cells; and administering the population of cells to the subject, wherein the method effectively increases the number of cells with characteristics of BAT cells in the subject.

We also describe a population of cells made by the methods described herein and pharmaceutical compositions comprising these populations of cells and pharmaceutically acceptable carriers.

We also describe cell delivery systems. These cell delivery systems include reservoirs containing one or more cells made by a method described herein, pharmaceutically acceptable carriers, and a delivery device, e.g., a needle or cannula, in fluid contact with the reservoir.

We further describe compositions comprising a population of thermogenic BMP-treated stem cell antigen-1 positive (Sca-1+), cells. In some instances, these cells are capable of non-shivering thermogenesis.

As used herein, "treatment" means any manner in which one or more of the symptoms of a disease or disorder are ameliorated or otherwise beneficially altered. As used herein, amelioration of the symptoms of a particular disorder refers to any lessening, whether permanent or temporary, lasting or transient, of the symptoms, which can be attributed to or associated with treatment by the compositions and methods of the present invention.

The terms "effective amount" and "effective to treat," as used herein, refer to an amount or a concentration of one or more of the compositions described herein utilized for a period of time (including acute or chronic administration and periodic or continuous administration) that is effective within the context of its administration for causing an intended effect or physiological outcome.

The term "subject" is used throughout the specification to describe an animal, human or non-human, rodent or non-rodent, to whom treatment according to the methods of the present invention is provided. Veterinary and non-veterinary applications are contemplated. The term includes, but is not limited to, mammals, e.g., humans, other primates, pigs, rodents such as mice and rats, rabbits, guinea pigs, hamsters, cows, horses, cats, dogs, sheep and goats. Typical subjects include humans, farm animals, and domestic pets such as cats and dogs. In some embodiments, the subject is a mammal. In some embodiments, the subject is a human subject, e.g., an obese human subject. In some embodiments, the subject is a non-human mammal, e.g., an experimental animal, a companion animal, or a food animal, e.g., a cow, pig, or sheep that is raised for food.

As used herein, an "isolated" or "purified" polypeptide, peptide, or protein is substantially free of cellular material or other contaminating proteins from the cell or tissue source from which the protein is derived, or substantially free from chemical precursors or other chemicals when chemically synthesized. "Substantially free" means that the preparation of a selected protein has less than about 30%, (e.g., less than 20%, 10%, or 5%) by dry weight, of non-selected protein or of chemical precursors. Such a non-selected protein is also referred to herein as "contaminating protein," When the isolated therapeutic proteins, peptides, or polypeptides are recombinantly produced, they can be substantially free of culture medium, i.e., culture medium represents less than about 20%, (e.g., less than about 10% or 5%) of the volume of the protein preparation.

As used herein, "obesity" refers to a disorder in a subject, wherein the subject's weight exceeds their ideal weight, according to standard tables, by 20% or more, e.g., 25%, 30%, 40%, and 50%, or more. Obese can also mean an individual with a body mass index (BMI) of 30 or more, e.g., 30-35 and 35-40 or more. For example, a subject diagnosed with class I obesity has a BMI range of 30-34.9. A subject with class II obesity has a BMI range of 35.0-39.9. A subject with class III obesity has a BMI greater than 40.

As used herein, "overweight" refers to a subject with a BMI range of 25.0-29.9.

As used herein, "stem cells" are capable of both self-renewal and differentiation into many different cell lineages (is pluripotent). "Progenitor cells" refers to a subset of stem cells with phenotypes similar to that of a stem cell. A progenitor cell is capable of self-renewal and is typically multipotent.

Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. The materials, methods, and examples are illustrative only and not intended to be limiting. In case of conflict, the present specification, including definitions, will control.

Other features and advantages of the invention will be apparent from the following detailed description and figures, and from the claims.

### BRIEF DESCRIPTION OF THE DRAWINGS

FIG. 1A is a collection of images of petridishes containing brown preadipocytes.
FIG. 1B is a collection of images of white preadipocytes. Cells were treated using the indicated bone morphogenic protein (BMP) and stained using Oil Red O to assess lipid accumulation. Control cells were not exposed to BMP. Lipid accumulation is shown by darkly stained cells, which appear red in color.
FIG. 2A is a bar graph showing uncoupling protein (UCP) and cell death-inducing DFF45-like effector A (CIDEA) expression in brown preadipocytes exposed to the indicated BMP as assessed using quantitative RT-PCR. Control cells were not exposed to BMP. *** P<0.001, ** P<0.01, * P<0.05.
FIG. 2B is an image of an immunoblot probed with antibodies specific for UCP-1 and ϑ-tubulin. ϑ-tubulin is shown as a loading control.
FIGs. 3A and 3B are images showing photographs Oil Red O stained control cells (A) and cells exposed to BMP-7 (B). Lipid accumulation is shown by darkly stained cells, which appear red in color. Lipid droplets are also clearly visible in both images.
FIG. 4A is a bar graph showing peroxisome proliferator activated receptor gamma (PPARK), fatty acid binding protein 4 (aP2), fatty acid synthase (FAS), CIDEA, and UCP-1 expression in control cells and cells exposed to BMP-7 as assessed using quantitative RT-PCR. Results are expressed as a percentage increase over the control cells.
FIG. 4B is a bar graph showing cAMP induced UCP-1 expression in control cells and cells exposed to BMP-7. The bar shown for UCP-1 treatment alone was used as the control bar for cAMP.
FIGs. 5A-5C are images of BMP7 treated Sca-1+ cells isolated from skeletal muscle that have been stained with Oil Red-O (ORO) to assess lipid accumulation. Control cells were not exposed to BMP. Lipid accumulation is shown by darkly stained cells, which appear red in color.
FIG. 6A is a scattergraph showing the population of Sca-1+/CD45-/Mac-1- cells obtained using FACS.
FIGs. 6B-6C are line graphs showing the presence or absence of various markers on the surface of Sca-1+/CD45-/Mac-1- cells as assessed using flow cytometry. Arrows indicate stained cells.
FIG. 7A-7N are line graphs showing the levels of markers detected over time using quantitative RT-PCR in cells exposed to BMP-7. Arrows indicate the result observed for cells exposed to BMP7.
FIG. 8 is a bar graph showing cAMP induced UCP-1 expression in control cells and cells exposed to BMP-7. The bar shown for UCP-1 treatment alone was used as the control bar for cAMP.
FIGs. 9A-F are a set of six photomicrophs. 9A, 9C, and 9E show Sca-1+ cells isolated from interscapular BAT (BAT)(9A), subcutaneous white adipose tissue (SQ-WAT)(9C), and visceral white adipose tissue (EPI-WAT)(9E) not treated with BMP7 (control). 9B shows cells isolated from interscapular BAT (BAT); 9D is cells from subcutaneous white adipose tissue (SQ-WAT), and 9F shows cells from visceral white adipose tissue (EPI-WAT) treated with BMP7 (BMP7).
FIGs. 10A-10B are bar graphs showing fatty acid synthase (FAS; A) and uncoupling protein-1 (UCP-1; B) expression levels in cells isolated from interscapular BAT (BAT), subcutaneous white adipose tissue (SQ-WAT), and visceral white adipose tissue (EPI-WAT).
FIG. 11 is a bar graph showing total Sca-1+ cells isolated from obesity resistant mice (129) and obesity prone mice (B6).
FIGs. 12A-12B are bar graphs showing UCP-1 and PPARK expression levels in BMP7 treated Sca-1+ cells isolated from obesity resistant mice (129) and obesity prone mice (B6). Untreated cells are shown as control.
FIGs. 13A-13D are fluorescent microscopic images. 13A and 13B show implanted BMP7 treated Sca-1+ green fluorescent protein cells 10 days post-implantation. Control images without implanted cells are shown in 13A and 13C. Implanted GFP cells are shown on the right (in Figs. 13B and 13D) appear as more darkly shaded cells.
FIGs. 14A-14F are images showing microscopic sections of fat pads into which BMP7 treated Sca-1+ green fluorescent protein cells were implanted. Sections are from non-green fluorescent protein-positive recipient mice. Green fluorescent protein-positive cells were detected by epifluorescence in recipient mice at 10 days after injection. Original magnification = 200X.
FIGs 15A-15F are bar graphs showing UCP-1, Cidea, PPARg, FAS, Myogenin and MyoD expression levels in muscle derived Sca-1+ cells treated with BMP-7, BMP-3, BMP-4 or vehicle control (C).

### DETAILED DESCRIPTION

The present disclosure provides, inter alia, compositions and methods useful for increasing BAT levels and/or function in a subject for treating subjects for obesity and weight related diseases and disorders such as the conditions noted below. These methods include promoting the differentiation of a defined subset of progenitor cells (e.g., progenitor cells capable of differentiating into a BAT cell) to or towards a BAT cell lineage. More specifically, the present disclosure is based, at least in part, on the discovery of a stem cell antigen-1 positive (Sca-1+) progenitor cell that when treated with bone morphogenic protein (BMP) is capable of differentiating to or towards a BAT cell lineage. As described herein, these compositions and methods can be used to increase the BAT cell number and/or function and/or to increase the ratio of BAT:WAT cells and thereby treat obesity and obesity related diseases and disorders in a subject.

Some of the methods described herein include implanting BMP-treated Sca-1+ progenitor cells that have been treated with an agent that increases BMP signaling. In general, the methods include treating (e.g., contacting) progenitor cells, e.g., Sca-1+ progenitor cells, with the compound in an amount sufficient to increase BMP signaling, and thereafter implanting the BMP activated Sca-1+ cells (e.g., at least one cell or a population of such cells) in a subject. Suitable agents can include the BMPs themselves, e.g., recombinant proteins, or nucleic acids that encode the BMPs, and/or agents that increase BMP signaling, e.g., small molecules, antibodies and antibody fragments, pharmaceutical agents, and other biological agents. In some embodiments, treating the cells includes genetically engineering the cells *in vitro* to express a BMP 2, -4, -5, -6, and/or -7 polypeptide. The cells are then administered to a subject. Populations of such genetically engineered Sca-1+ progenitor cells are also included within the scope of the present invention. Other compounds are described herein.

### Cell Types

Cells suitable for use in the methods described herein include stem cell antigen-1 positive (Sca-1+) progenitor cells, for example mammalian Sca-1+ progenitor cells. Sca-1 is an 18-kDa glycosyl phosphatidylinositol-anchored surface protein, which serves as a stem cell marker for both hematopoetic stem cells and tissue resident progenitor cells. Sca-1+ progenitor cells useful in the present disclosure can be isolated from a variety of tissues and organs including, but not limited to, for example, skeletal muscle, prostate, dermis, the cardiovascular system, mammary gland, liver, neonatal skin, calvaria, bone marrow, the intestine, and adipose tissue (e.g., adipose tissue deposits). In some embodiments, Sca-1+ cells are isolated from skeletal muscle, e.g., skeletal muscle bundles. In some embodiments, Sca-1+ cells are isolated from adipose tissue, e.g. adipose depots.

Sca-1+ progenitor cells can be unipotent, multipotent, and pluruipotent and can be of mesenchymal origin. In some embodiments, Sca-1+ progenitor cells are non-myogenic progenitor cells. In some embodiments, Sca-1+ progenitor cells can be a pluripotent stem cell artificially derived (e.g., differentiated or partially differentiated) from a non-pluripotent cell. Such cells are know in the art as induced pluripotent stem cells, which is commonly abbreviated to iPS or iPSCs (for a review see, e.g., Nishikawa et al., Mol. Cell. Biol., 9:725-729,2008).

Sca-1+ progenitor cells can be identified by determining the presence or absence of one or more cell surface expression markers. Exemplary cell surface markers that can be used to identify a Sca-1+ progenitor cells include, but are not limited to, Sca-1, CD45, Mac-1, CD29 (integrin ϑ1), CD105 (Endoglin), CD166 (ALCAM), desmin, vimentin, and c-kit.

In some embodiments, a Sca-1+ positive cell can be identified by detecting Sca-1. Alternatively or in addition, Sca-1+ progenitor cells can be identified by detecting additional cell surface markers. In some embodiments, Sca-1+ progenitor cells useful in the methods described herein are negative for the cell surface markers CD45 and/or Mac-1, e.g., cells can be Sca-1+/CD45-, Sca-1+/Mac-1, or Sca-1+/Cd45-/Mac-1-. In some embodiments, Sca-1+ progenitor cells can be Sca-1+/CD29+ cells. In some embodiments, Sca-1+ progenitor cells can be Sca-1+/CD29+/Cd45-/Mac-1- cells. In some embodiments, Sca-1+ progenitor cells can be Sca-1+ cells with one or more of the following cell surface markers CD29+, CD34+, Cd45-, Mac-1-, and CD117-.

In some embodiments, additional cell surface markers can be identified on the surface of a Sca-1+ progenitor cell following identification of the Sca-1+ progenitor cell, e.g., to further characterize and/or confirm the identity of the cell line. For example, Sca-1+ cells can be assessed to determine the cell surface expression of other markers including but not limited to, for example, CD29 (integrin ϑ1), CD105 (Endoglin), CD166 (ALCAM), desmin, vimentin, CD34, CD133, and c-kit.

Once a population of cells is identified with a defined cell surface marker (e.g., Sca-1), the methods for identifying, isolating, and enriching a population of such cells are routine and known in the art. Such methods include for example, immunomagnetic cell sorting, fluorescence activated cell sorting (FACS), adherence to tissue culture plates and flasks, or culturing under conditions that favor the growth of the desired stem cells, and combinations thereof. Using such methods, a Sca-1+ progenitor cell or a population of Sca-1+ cells can isolated, purified, and/or enriched, e.g., a population of Sca-1+ progenitor cells in which at least 60%, e.g., 70%, 80%, 90% or more of the cells are Sca-1+ cells.

In some embodiments, a Sca-1+ cell or cells can be obtained without identifying, isolating, and/or enriching the cells. For example, Sca-1+ cells can be obtained from a cell depository, e.g., the American Type Culture Collection.

In some embodiments, an isolated Sca-1+ cell, clone, or population can be cultured using standard cell culture techniques in order to obtain a larger population of cells.

In some embodiments, Sca-1+ cells can be stored in liquid nitrogen. The techniques required for preparing cells for storage in liquid nitrogen and methods for storing such cells in liquid nitrogen are routine and are known in the art.

The term "primary cell" includes cells present in a suspension of cells isolated from a mammalian tissue source (prior to their being plated, i.e., attached to a tissue culture substrate such as a dish or flask), cells present in an explant derived from tissue, both of the previous types of cells plated for the first time, and cell suspensions derived from these plated cells. The term "secondary cell" or "cell strain" refers to cells at all subsequent steps in culturing. Secondary cells are cell strains that consist of secondary cells that have been passaged one or more times.

Primary and secondary stem cells can be obtained from a variety of tissues and include cell types which can be maintained and propagated in culture. Primary cells are preferably obtained from the individual or animal to whom the BMP-treated cells are administered. However, primary cells can also be obtained from a donor (e.g., an individual other than the recipient, typically of the same species, preferably an immunologically compatible individual). Methods for obtaining and culturing such cells are known in the art.

The methods can include allowing Sca-1+ progenitor cells to undergo sufficient rounds of doubling, e.g., to produce either a clonal cell strain or a heterogeneous cell strain of desired size, e.g., a sufficient number to provide a therapeutic effect to an individual, or a sufficient number to establish a stable cell line, before or after BMP-activation. Where the cells are not transfected but rather treated with a BMP, the cells can be cultured for a time in the absence of the BMP, then in the presence of the BMP for a time (e.g., 1, 2, 3 or more days) before implantation into the subject. The cells can be washed (e.g., in isotonic PBS) before implantation to remove any contaminants, including BMPs or components of growth media, before implantation. The number of required cells is variable and depends on a variety of factors, including but not limited to, the use of the transfected cells, the functional level of the exogenous DNA in the transfected cells, the site of implantation of the transfected cells (for example, the number of cells that can be used is limited by the anatomical site of implantation), and the age, surface area, and clinical condition of the subject. In some embodiments, the population of BMP-treated stem cells includes at least 10⁷, 10⁸, 10⁹, or more cells.

BMP-treated Sca-1+ cells are Sca-1+ cells that have an enhanced level of BMP signaling, e.g., BMP-2, -4, -5, -6, and/or -7 signaling, wherein the level of BMP signaling has been increased by direct human intervention. BMP signaling can be enhanced in the cells by any method known in the art or described herein, e.g., by treating the cell with a compound that enhances BMP signaling as described herein, e.g., a BMP polypeptide or nucleic acid. Populations of stem cells activated by methods described herein are also included within the present invention. Optionally, the population of BMP-treated cells can be suspended in a pharmaceutically acceptable carrier, e.g., for storage or implantation. As used herein, the language "pharmaceutically acceptable carrier" is intended to include any and all solvents, media, antibacterial and antifungal agents, isotonic agents, and the like, compatible with pharmaceutical administration and viability of the cells. In general, the cells will be maintained in a sterile state. The use of such media and agents for pharmaceutically active substances are known. Except insofar as any conventional media or agent is incompatible with the active compound, such media can be used in the compositions of the invention. Supplementary active compounds can also be incorporated into the compositions.

The cells can be autologous, allogeneic, or xenogeneic. In some embodiments, methods described herein can include obtaining a population of stem cells from a subject, optionally culturing and/or enriching the stem cells to obtain a purified population of stem cells, treating the cells with an agent that enhances BMP signaling as described herein to activate the cells, and implanting the cells in the same subject from which they were removed. In some embodiments, the cells are allogeneic or xenogeneic; if necessary, immune suppression can be administered to prevent rejection of the cells.

In some embodiments, the Sca-1+ progenitor cells used in the methods and compositions described herein express one or more BMP receptors, e.g., type I or II BMP receptors.

### Bone Morphogenic Proteins (BMPs)

In some embodiments, of the methods described herein compounds that enhance BMP signaling are full length or truncated BMPs (e.g., BMPs, polypeptides, and peptides).

BMPs are members of the transforming growth factor-β superfamily that are involved in multiple key steps of embryonic development as well as throughout life (Kishigami and Mishina, Cytokine.Growth Factor.Rev. 16:265-278 (2005); Chen et al., Growth Factors. 22:233-241 (2004); Yamamoto and Oelgeschlager, Naturwissenschaften 91:519-534 (2004)). BMPs have been shown to play a role in two different stages of adipocyte development. First, BMP-2 and 4 stimulate differentiation of multipotent mesenchymal cells and bone marrow stromal cells into adipocytes under appropriate conditions (Butterwith et al., Biochem.Soc Trans 24:163S (1996); Chen et al., J. Cell. Biol. 142:295-305 (1998); Chen et al., J Cell Biochem. 82:187-199 (2001); Tang et al., Proc Natl.Acad Sci U S.A. 101:9607-9611 (2004)). In addition, BMPs also stimulate the differentiation of committed white preadipocytes (Sottile and Seuwen, FEBS Lett. 475:201-204 (2000); Rebbapragada et al., Mol. Cell. Biol. 23:7230-7242 (2003)). However, other studies showed that BMP-2 suppressed adipogenic differentiation and promoted osteogenesis in multipotent mesenchymal progenitors via homeobox gene, Msx2 (Ichida et al., J. Biol. Chem. 279:34015-34022 (2004); S. L. Cheng et al., J. Biol. Chem. 278:45969-45977 (2003)).

BMPs bind to specific type-I and -II serine/threonine kinase receptor complexes, RIa, RIb, and RII, which signal through SMAD proteins or the p38 mitogen-activated protein kinase (MAPK). The BMPs are important regulators of key events in many aspects of tissue development and morphogenesis, including the processes of bone formation during embryogenesis, postnatal growth, remodeling and regeneration of the skeleton. Localization studies in both human and mouse tissues have demonstrated high levels of mRNA expression and protein synthesis for various BMPs in adipose, heart, lung, small intestine, limb bud and teeth.

BMPs have been used in the clinic in the treatment of bone and cartilage disorders or wounds. The effective clinical use of recombinant BMPs is discussed in Einhorn, J. Bone and Joint Surgery 85A:82-88 (2003), and Sandhu, Spine 28(15):S64-73 (2003). A BMP polypeptide (e.g., a mature BMP polypeptide) is itself is a viable therapeutic compound because BMPs are small secreted proteins that are internalized into their target cells where they exert their activity. Although the human proteins are described herein, one of skill in the art will appreciate that when another species is the intended recipient of the treated cells, homologous proteins from that species can also be used, e.g., cow, pig, sheep, or goat. Such homologous proteins can be identified, e.g., using methods known in the art, e.g., searching available databases for homologs identified in the target species, e.g., the homologene database.

As described herein, BMP-2, -4, -5, -6, and -7 are involved in brown adipocyte differentiation, and treatment of Sca-1+ progenitor cells with BMP-2, -4, -5, -6, and/or -7 promotes brown adipogenesis. BMP-2, -4, -5, -6, and/or -7 are thus therapeutic, diagnostic and drug discovery targets for adipose-related disorders, such as obesity and related disorders such as diabetes, insulin resistance, hyperglycemia, hyperlipidemia, and hypercholesterolemia. In general, the methods described herein include implanting a population of BMP-treated Sca-1+cells as described herein into a subject.

BMPs suitable for use in the methods described herein include:

### BMP-2

BMP-2 is 396 amino acids in length, localized to chromosome 20p12 in human. The nucleotide and amino acid sequences of human BMP-2 are disclosed in Wozney et al., Science 242(4885):1528-1534 (1988). BMP2 belongs to the transforming growth factor-beta (TGFβ) superfamily. Bone morphogenetic protein induces bone formation, and BMP2 is a candidate gene for the autosomal dominant disease of fibrodysplasia (myositis) ossificans progressive. Bone morphogenetic protein 2 regulates myogenesis through dosage-dependent PAX3 expression in pre-myogenic cells, and is expressed in mesoderm under SHM control through the SOX9.

The human BMP-2 is shown below. Amino acids 38-268 are the TGFβ propeptide domain, and 291-396 are the TGFβ family N-terminal domain. Amino acids 283-396 are the mature peptide. The sequence is set forth in Wozney et al., Science 242:1528-1534 (1988).

1 MVAGTRCLLA LLLPQVLLGG AAGLVPELGR RKFAAASSGR PSSQPSDEVL SEFELRLLSM
61 FGLKQRPTPS RDAVVPPYML DLYRRHSGQP GSPAPDHRLE RAASRANTVR SFHHEESLEE
121 LPETSGKTTR RFFFNLSSIP TEEFITSAEL QVFREQMQDA LGNNSSFHHR INIYEIIKPA
181 TANSKFPVTR LLDTRLVNQN ASRWESFDVT PAVMRWTAQG HANHGFVVEV AHLEEKQGVS
241 KRHVRISRSL HQDEHSWSQI RPLLVTFGHD GKGHPLHKRE KRQAKHKQRK RLKSSCKRHP
301 LYVDFSDVGW NDWIVAPPGY HAFYCHGECP FPLADHLNST NHAIVQTLVN SVNSKIPKAC
361 CVPTELSAIS MLYLDENEKV VLKNYQDMVV EGCGCR (SEQ ID NO: 1)

The mature form of BMP-2 contains four potential N-linked glycosylation sites per polypeptide chain, and four potential disulfide bridges. See UniProt entry No. P12643; HomoloGene:926.

### BMP-4

BMP-4 induces cartilage and bone formation, and is important in mesoderm induction, tooth development, limb formation and fracture repair. The sequence of the BMP-4 preproprotein is shown below. Amino acids 41-276 are the TGFβ propeptide domain, and 302-408 are the TGFβ family N-terminal domain. Amino acids 293-408 are the mature peptide. The sequence is set forth in Wozney et al., Science 242:1528-1534 (1988).

1 MIPGNRMLMV VLLCQVLLGG ASHASLIPET GKKKVAEIQG HAGGRRSGQS HELLRDFEAT
61 LLQMFGLRRR PQPSKSAVIP DYMRDLYRLQ SGEEEEEQIH STGLEYPERP ASRANTVRSF
121 HHEEHLENIP GTSENSAFRF LFNLSSIPEN EAISSAELRL FREQVDQGPD WERGFHRINI
181 YEVMKPPAEV VPGHLITRLL DTRLVHHNVT RWETFDVSPA VLRWTREKQP NYGLAIEVTH
241 LHQTRTHQGQ HVRISRSLPQ GSGNWAQLRP LLVTFGHDGR GHALTRRRRA KRSPKHHSQR
301 ARKKNKNCRR HSLYVDFSDV GWNDWIVAPP GYQAFYCHGD CPFPLADHLN STNHAIVQTL
361 VNSVNSSIPK ACCVPTELSA ISMLYLDEYD KVVLKNYQEM VVEGCGCR (SEQ ID
NO:2)

The mature form of BMP-4 contains four potential N-linked glycosylation sites per polypeptide chain. A variant exists in which V152 is an A. See UniProt Accession No. P12644; HomoloGene:7247.

### BMP-5

The BMP-5 preproprotein is a 454 amino acid protein, as shown below. BMP-5 induces cartilage and bone formation. The sequence is set forth in Celeste et al., Proc. Natl. Acad. Sci. U.S.A., 87, 9843-9847, 1990.

1 MHLTVFLLKG IVGFLWSCWV LVGYAKGGLG DNHVHSSFIY RRLRNHERRE IQREILSILG
61 LPHRPRPFSP GKQASSAPLF MLDLYNAMTN EENPEESEYS VRASLAEETR GARKGYPASP
121 NGYPRRIQLS RTTPLTTQSP PLASLHDTNF LNDADMVMSF VNLVERDKDF SHQRRHYKEF
181 RFDLTQIPHG EAVTAAEFRI YKDRSNNRFE NETIKISIYQ IIKEYTNRDA DLFLLDTRKA
241 QALDVGWLVF DITVTSNHWV INPQNNLGLQ LCAETGDGRS INVKSAGLVG RQGPQSKQPF
301 MVAFFKASEV LLRSVRAANK RKNQNRNKSS SHQDSSRMSS VGDYNTSEQK QACKKHELYV
361 SFRDLGWQDW IIAPEGYAAF YCDGECSFPL NAHMNATNHA IVQTLVHLMF PDHVPKPCCA
421 PTKLNAISVL YFDDSSNVIL KKYRNMVVRS CGCH (SEQ ID NO:3)

The mature BMP-5 protein is believed to be amino acids 323-454 of SEQ ID NO:3, and has four potential N-linked glycosylation sites per polypeptide chain, and four potential disulfide bridges. See UniProt Accession Nos. P22003; Q9H547; or Q9NTM5; HomoloGene:22412.

### BMP-6

BMP-6 is an autocrine stimulator of chondrocyte differentiation, and is involved in the development of embryonic neural, and urinary systems, as well as growth and differentiation of liver and keratinocytes. BMP-6 knockout mice are viable and show a slight delay in ossification of the sternum. BMP-6 (precursor) is a 57 kD protein, 513 amino acids in length, localized to chromosome 6p24 in human. The nucleotide and amino acid sequence of human BMP-6 is disclosed in U.S. Patent 5,187,076. BMP-6 is predicted to be synthesized as a precursor molecule which is cleaved to yield a 132 amino acid mature polypeptide with a calculated molecular weight of approximately 15 Kd. The mature form of BMP-6 contains three potential N-linked glycosylation sites per polypeptide chain. The active BMP-6 protein molecule is likely a dimer. Processing of BMP-6 into the mature form involves dimerization and removal of the N-terminal region in a manner analogous to the processing of the related protein TGFϑ (Gentry et al., Molec. Cell. Biol. 8:4162 (1988); Dernyck et al., Nature 316:701 (1985)). The human BMP-6 precursor is shown below. The mature polypeptide is believed to include amino acids 374-513 of SEQ ID NO:4. Other active BMP-6 polypeptides include polypeptides including amino acids 382-513, 388-513 and 412-513 of SEQ ID NO:4.

 61
 121
 181
 241
 301
 361
 421
 481
 (SEQ ID NO:4)

The human BMP-6 promoter has been characterized (See Tamada et al., Biochim Biophys Acta. 1998, 1395(3):247-51), and can be used in methods described herein. See UniProt Accession No. P22004; HomoloGene:1300.

Administration, antisense treatment, and quantitation of BMP-6 are described in Boden et al. (Endocrinology Vol. 138, No. 7 2820-2828).

### BMP-7

BMP-7 also belongs to the TGFβ superfamily. It induces cartilage and bone formation, and may be the osteoinductive factor responsible for the phenomenon of epithelial osteogenesis. BMP-7 plays a role in calcium regulation and bone homeostasis, and in the regulation of anti-inflammatory response in the adult gut tissue. The sequence of BMP-7 is shown below:

  1 MHVRSLRAAA PHSFVALWAP LFLLRSALAD FSLDNEVHSS FIHRRLRSQE RREMQREILS
 61 ILGLPHRPRP HLQGKHNSAP MFMLDLYNAM AVEEGGGPGG QGFSYPYKAV FSTQGPPLAS
121 LQDSHFLTDA DMVMSFVNLV EHDKEFFHPR YHHREFRFDL SKIPEGEAVT AAEFRIYKDY
181 IRERFDNETF RISVYQVLQE HLGRESDLFL LDSRTLWASE EGWLVFDITA TSNHWVVNPR
241 HNLGLQLSVE TLDGQSINPK LAGLIGRHGP QNKQPFMVAF FKATEVHFRS IRSTGSKQRS
301 QNRSKTPKNQ EALRMANVAE NSSSDQRQAC KKHELYVSFR DLGWQDWIIA PEGYAAYYCE
361 GECAFPLNSY MNATNHAIVQ TLVHFINPET VPKPCCAPTQ LNAISVLYFD DSSNVILKKY
421 RNMVVRACGC H  (SEQ ID NO:5)

Amino acids 1-29 are a potential signal sequence; 30-431 are the prepropeptide, and 293-431 are the mature protein. The mature form of BMP-7 contains four potential N-linked glycosylation sites per polypeptide chain, and four potential disulfide bridges. See UniProt Accession No. P18075; HomoloGene:20410.

The compositions and methods described herein also contemplate the use of amino acid sequences with at least 50% (e.g., at least 60%, 70%, 80%, 90%, 95%, 98%, and 99%) identity to one or more of the above shown BMP sequences. As used herein, such sequences are also referred to as BMPs.

To determine the percent identity of two sequences, the sequences are aligned for optimal comparison purposes (gaps are introduced in one or both of a first and a second amino acid or nucleic acid sequence as required for optimal alignment, and non-homologous sequences can be disregarded for comparison purposes). The length of a reference sequence aligned for comparison purposes is at least 80% (in some embodiments, about 85%, 90%, 95%, or 100% of the length of the reference sequence) is aligned. The nucleotides or residues at corresponding positions are then compared. When a position in the first sequence is occupied by the same nucleotide or residue as the corresponding position in the second sequence, then the molecules are identical at that position. The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. For example, the percent identity between two amino acid sequences can be determined using the Needleman and Wunsch ((1970) J. Mol. Biol. 48:444-453) algorithm which has been incorporated into the GAP program in the GCG software package, using a Blossum 62 scoring matrix with a gap penalty of 12, a gap extend penalty of 4, and a frameshift gap penalty of 5.

BMPs can be generated using recombinant techniques or using chemical synthesis. Methods for generating such polypeptides, and methods required for the purification of such polypeptides, are known in the art, see, e.g. Sambrook and Russel, Molecular Cloning; A laboratory Mannual (CSHL Press, 3rtd Edition, 2001).

Modifications can be made to a BMP to alter the pharmacokinetic properties of the protein. For example, such modifications can result in longer circulatory half-life, an increase in cellular uptake, improved distribution to targeted tissues, a decrease in clearance and/or a decrease of immunogenicity. A number of approaches useful to optimize the therapeutic activity of a BMP are known in the art, including chemical modification.

### Expression Systems

BMPs can be expressed as recombinant proteins. For recombinant proteins, the choice of expression system can influence pharmacokinetic characteristics. Differences in post-translational processing between expression systems can lead to recombinant proteins of varying molecular size and charge, which can affect circulatory half-life, rate of clearance and immunogenicity, for example. The pharmacokinetic properties of the protein may be optimized by the appropriate selection of an expression system, such as selection of a bacterial, viral, or mammalian expression system. Exemplary mammalian cell lines useful in expression systems for therapeutic proteins are Chinese hamster ovary, (CHO) cells, the monkey COS-1 cell line and the CV-1 cell line.

The recombinant expression vectors of the invention can be designed for expression of BMPs in prokaryotic or eukaryotic cells. For example, proteins of the invention can be expressed in *E. coli,* insect cells (e.g., using baculovirus expression vectors), yeast cells or mammalian cells. Suitable host cells are discussed further in Goeddel, Gene Expression Technology: Methods in Enzymology, 185, (Academic Press, San Diego, CA 1990). Alternatively, the recombinant expression vector can be transcribed and translated *in vitro,* for example using T7 promoter regulatory sequences and T7 polymerase.

### Chemical Modification

BMPs can be chemically altered to enhance the pharmacokinetic properties, while maintaining activity. The protein can be covalently linked to a variety of moieties, altering the molecular size and charge of the protein and consequently its pharmacokinetic characteristics. The moieties are preferably non-toxic and biocompatible. In one embodiment, poly-ethylene glycol (PEG) can be covalently attached to the protein (PEGylation). A variety of PEG molecules are known and/or commercially available (See, e.g., Sigma-Aldrich catalog). PEGylation can increase the stability of the protein, decrease immunogenicity by steric masking of epitopes, and improve half-life by decreasing glomerular filtration. (See, e.g., Harris and Zalipsky, Poly(ethylene glycol): Chemistry and Biological Applications, ACS Symposium Series, No. 680, American Chemical Society (1997); Harris et al., Clinical Pharmacokinetics, 40: 485-563, 2001). Examples of therapeutic proteins administered as PEG constructs include Adagen™ (PEG-ADA) and Oncospar™ (Pegylated asparaginase). In another embodiment, the protein can be similarly linked to oxidized dextrans via an amino group. (See Sheffield, Curr. Drug Targets Cardiovas. Haemat. Dis., 1:1-22, 2001). In yet another embodiment, conjugation of arginine oligomers to cyclosporin A can facilitates topical delivery (Rothbard et al., Nat. Med., 6: 1253-1257, 2000).

Furthermore, the protein can be chemically linked to another protein, e.g., crosslinked (via a bifunctional cross-linking reagent, for example) to a carrier protein to form a larger molecular weight complex with longer circulatory half-life and improved cellular uptake. In some embodiments, the carrier protein can be a serum protein, such as albumin. In another embodiment, the therapeutic protein can cross-link with itself to form a homodimer, a trimer, or a higher analog, e.g., via heterobifunctional or homobifunctional cross-linking reagents (see Stykowski et al., Proc. Natl. Acad. Sci. USA, 95:1184-1188, 1998). Increasing the molecular weight and size of the therapeutic protein through dimerization or trimerization can decrease clearance.

### Protein Formulations

The formulation of the protein may also be optimized. For example, BMPs can be formulated in a carrier system. The carrier can be a colloidal system. The colloidal system can be a liposome, a phospholipid bilayer vehicle. In one embodiment, the therapeutic protein is encapsulated in a liposome while maintaining protein integrity. As one skilled in the art would appreciate, there are a variety of methods to prepare liposomes. (See Lichtenberg et al., Methods Biochem. Anal., 33:337-462, 1988; Anselem et al., Liposome Technology, CRC Press (1993)). Liposomal formulations can delay clearance and increase cellular uptake (See Reddy, Ann. Pharmacother., 34:915-923, 2000).

The carrier can also be a polymer, e.g., a biodegradable, biocompatible polymer matrix. In one embodiment, the therapeutic protein can be embedded in the polymer matrix, while maintaining protein integrity. The polymer may be natural, such as polypeptides, proteins or polysaccharides, or synthetic, such as poly(I-hydroxy) acids. Examples include carriers made of, e.g., collagen, fibronectin, elastin, cellulose acetate, cellulose nitrate, polysaccharide, fibrin, gelatin, and combinations thereof. In one embodiment, the polymer is poly-lactic acid (PLA) or copoly lactic/glycolic acid (PGLA). The polymeric matrices can be prepared and isolated in a variety of forms and sizes, including microspheres and nanospheres. Polymer formulations can lead to prolonged duration of therapeutic effect. (See Reddy, Ann. Pharmacother., 34:915-923, 2000). A polymer formulation for human growth hormone (hGH) has been used in clinical trials. (See Kozarich and Rich, Chemical Biology, 2:548-552, 1998).

Examples of polymer microsphere sustained release formulations are described in PCT publication WO 99/15154 (Tracy et al.), U.S Pat. Nos. 5,674,534 and 5,716,644 (both to Zale et al.), PCT publication WO 96/40073 (Zale et al.), and PCT publication WO 00/38651 (Shah et al.). U.S. Pat. Nos. 5,674,534 and 5,716,644 and PCT publication WO 96/40073 describe a polymeric matrix containing particles of erythropoietin that are stabilized against aggregation with a salt.

In some embodiments, the fusion protein includes a cell-penetrating peptide sequence that facilitates entry of BMPs into a cell, e.g., HIV-derived TAT peptide, penetratins, transportans, or hCT derived cell-penetrating peptides, see, e.g., Caron et al., Mol Ther. 3:310-8, 2001; Langel, Cell-Penetrating Peptides: Processes and Applications (CRC Press, Boca Raton FL 2002); El-Andaloussi et al., Curr. Pharm. Des., 11:3597-611, 2005; and Deshayes et al., Cell. Mol. Life Sci., 62:1839-49, 2005.

### Peptide mimetics

In some embodiments of the methods described herein the BMP protein is a peptide mimetic (e.g., either a peptide or nonpeptide peptide mimetic). Synthesis of nonpeptide compounds that mimic peptide sequences is known in the art. Such peptide mimetics include BMPs that can be modified according to methods known in the art for producing peptidomimetics. See, e.g., Kazmierski, W.M., ed., Peptidomimetics Protocols, Human Press (Totowa NJ 1998); Goodman et al., eds., Houben-Weyl Methods of Organic Chemistry: Synthesis of Peptides and Peptidomimetics, Thiele Verlag (New York 2003); and Mayo et al., J. Biol. Chem., 278:45746, 2003. In some cases, these modified peptidomimetic versions of the peptides and fragments disclosed herein exhibit enhanced stability *in vivo,* relative to the non-peptidomimetic peptides. Methods for creating a peptidomimetic include substituting one or more, e.g., all, of the amino acids in a peptide sequence with D-amino acid enantiomers. Such sequences are referred to herein as "retro" sequences. In another method, the N-terminal to C-terminal order of the amino acid residues is reversed, such that the order of amino acid residues from the N-terminus to the C-terminus of the original peptide becomes the order of amino acid residues from the C-terminus to the N-terminus in the modified peptidomimetic. Such sequences can be referred to as "inverso" sequences. Peptidomimetics can be both the retro and inverso versions, i.e., the "retro-inverso" version of a peptide disclosed herein. The new peptidomimetics can be composed of D-amino acids arranged so that the order of amino acid residues from the N-terminus to the C-terminus in the peptidomimetic corresponds to the order of amino acid residues from the C-terminus to the N-terminus in the original peptide.

Other methods for making a peptidomimetics include replacing one or more amino acid residues in a peptide with a chemically distinct but recognized functional analog of the amino acid, i.e., an artificial amino acid analog. Artificial amino acid analogs include ϑ-amino acids, ϑ-substituted ϑ-amino acids ("ϑ³-amino acids"), phosphorous analogs of amino acids, such as ∀-amino phosphonic acids and ∀-amino phosphinic acids, and amino acids having non-peptide linkages. Artificial amino acids can be used to create peptidomimetics, such as peptoid oligomers (e.g., peptoid amide or ester analogues), ϑ-peptides, cyclic peptides, oligourea or oligocarbamate peptides; or heterocyclic ring molecules. These sequences can be modified, e.g., by biotinylation of the amino terminus and amidation of the carboxy terminus.

Any of the peptides described herein, including the variant forms described herein, can further include a heterologous polypeptide. The heterologous polypeptide can be a polypeptide that increases the circulating half-life of the peptide to which it is attached (*e.g*., fused, as in a fusion protein). The heterologous polypeptide can be an albumin (*e.g*., a human serum albumin or a portion thereof) or a portion of an immunoglobulin (*e.g.,* the Fc region of an IgG). The heterologous polypeptide can be a mitochondrial-penetrating moiety.

The present disclosure also contemplates synthetic mimicking compounds. As is known in the art, peptides can be synthesized by linking an amino group to a carboxyl group that has been activated by reaction with a coupling agent, such as dicyclohexylcarbodiimide (DCC). The attack of a free amino group on the activated carboxyl leads to the formation of a peptide bond and the release of dicyclohexylurea. It can be necessary to protect potentially reactive groups other than the amino and carboxyl groups intended to react. For example, the (α-amino group of the component containing the activated carboxyl group can be blocked with a tertbutyloxycarbonyl group. This protecting group can be subsequently removed by exposing the peptide to dilute acid, which leaves peptide bonds intact. With this method, peptides can be readily synthesized by a solid phase method by adding amino acids stepwise to a growing peptide chain that is linked to an insoluble matrix, such as polystyrene beads. The carboxyl-terminal amino acid (with an amino protecting group) of the desired peptide sequence is first anchored to the polystyrene beads. The protecting group of the amino acid is then removed. The next amino acid (with the protecting group) is added with the coupling agent. This is followed by a washing cycle. The cycle is repeated as necessary.

In some embodiments, the mimetics of the present disclosure are peptides having sequence homology to the herein-described BMP protein. These mimetics include, but are not limited to, peptides in which L-amino acids are replaced by their D-isomers. One common methodology for evaluating sequence homology, and more importantly statistically significant similarities, is to use a Monte Carlo analysis using an algorithm written by Lipman and Pearson to obtain a Z value. According to this analysis, a Z value greater than 6 indicates probable significance, and a Z value greater than 10 is considered to be statistically significant (Pearson and Lipman, Proc. Natl. Acad. Sci. (USA), 85:2444-2448, 1988; Lipman and Pearson, Science, 227:1435-1441, 1985. More generally, the BMPs described herein and the mimetics described above can be synthesized using any known methods, including tea-bag methodology or solid phase peptide synthesis procedures described by Merrifield et al., Biochemistry, 21:5020-5031, 1982; Houghten Wellings, Proc. Natl. Acad. Sci. (USA), 82:5131-5135, 1985; Atherton, Methods in Enzymology, 289:44-66, 1997, or Guy and Fields, Methods in Enzymology, 289:67-83, 1997, or using a commercially available automated synthesizer.

### Targeted Peptides

In some embodiments, BMPs and/or BMP agonists can be targeted to a Sca-1 + cell in vitro and/or in vivo. Methods for targeting compounds against specific cell types and tissues are known in the art. For example, compositions and methods for targeting peptides and other therapeutic agents to specific cell or tissues include the use of materials that can target antigens or markers that are unique or specific to the intended target cell or tissue, for example, including but not limited to, antibodies or antigen binding fragments of antibodies, and short affinity peptides. In some embodiments, such materials can be specific for Sca-1 and/or CD29.

As one example, nanoparticles linked to a BMP or BMP agonist and to Sca-1 targeting moieties, e.g., anti-Sca-1 antibodies or antigen-binding fragments thereof, can be used. The BMP and targeting moiety can be on the same or on different nanoparticles. In some embodiments, the composition includes a BMP and a separate targeting moiety, e.g., a nanoparticle linked to a Sca-1 targeting moiety.

A number of biocompatible nanoparticles are known in the art, e.g., organic or inorganic nanoparticles. Liposomes, dendrimers, carbon nanomaterials and polymeric micelles are examples of organic nanoparticles. Quantum dots can also be used. Inorganic nanoparticles include metallic nanoparticle, e.g., Au, Ni, Pt and TiO₂ nanoparticles. Magnetic nanoparticles can also be used, e.g., spherical nanocrystals of 10-20 nm with a Fe²⁺ and/or Fe³⁺ core surrounded by dextran or PEG molecules. In some embodiments, colloidal gold nanoparticles are used, e.g., as described in Qian et al., Nat. Biotechnol. 26(1):83-90 (2008); U.S. Pat. Nos. 7060121; 7232474; and U.S. P.G. Pub. No. 2008/0166706. Suitable nanoparticles, and methods for constructing and using multifunctional nanoparticles, are discussed in e.g., Sanvicens and Marco, Trends Biotech., 26(8): 425-433 (2008).

In all embodiments, the nanoparticles are attached (linked) to the BMPs and targeting moieties described herein via a functional groups. In some embodiments, the nanoparticles are associated with a polymer that includes the functional groups, and also serves to keep the metal oxides dispersed from each other. The polymer can be a synthetic polymer, such as, but not limited to, polyethylene glycol or silane, natural polymers, or derivatives of either synthetic or natural polymers or a combination of these. Useful polymers are hydrophilic. In some embodiments, the polymer "coating" is not a continuous film around the magnetic metal oxide, but is a "mesh" or "cloud" of extended polymer chains attached to and surrounding the metal oxide. The polymer can comprise polysaccharides and derivatives, including dextran, pullanan, carboxydextran, carboxmethyl dextran, and/or reduced carboxymethyl dextran. The metal oxide can be a collection of one or more crystals that contact each other, or that are individually entrapped or surrounded by the polymer.

In other embodiments, the nanoparticles are associated with non-polymeric functional group compositions. Methods are known to synthesize stabilized, functionalized nanoparticles without associated polymers, which are also within the scope of this invention. Such methods are described, for example, in Halbreich et al., Biochimie, 80 (5-6):379-90, 1998.

In some embodiments, the nanoparticles have an overall size of less than about 1-100 nm, e.g., about 25-75 nm, e.g., about 40-60 nm, or about 50-60 nm in diameter. The polymer component in some embodiments can be in the form of a coating, e.g., about 5 to 20 nm thick or more. The overall size of the nanoparticles is about 15 to 200 nm, e.g., about 20 to 100 nm, about 40 to 60 nm; or about 60 nm.

### Antibodies

In some embodiments, BMPs can be targeted to a Sca-1+ cell or tissue comprising Sca-1+ cells using a antibody, e.g., to selectively target a cell.

In some embodiments, the BMP protein can be substituted for or used in conjunction with an antibody that increases the expression and/or activity of one or more BMPs, e.g., in a cell.

The term "antibody," as used herein, refers to full-length, two-chain immunoglobulin molecules and antigen-binding portions and fragments thereof, including synthetic variants. A typical full-length antibody includes two heavy (H) chain variable regions (abbreviated herein as VH), and two light (L) chain variable regions (abbreviated herein as VL). The term "antigen-binding fragment" of an antibody, as used herein, refers to one or more fragments of a full-length antibody that retain the ability to specifically bind to a target. Examples of antigen-binding fragments include, but are not limited to: (i) a Fab fragment, a monovalent fragment consisting of the VL, VH, CL and CH1 domains; (ii) a F(ab')₂ fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., Nature 341:544-546 (1989)), which consists of a VH domain; and (vi) an isolated complementarity determining region (CDR). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see e.g., Bird et al. Science 242:423-426, 1988; and Huston et al. Proc. Natl. Acad. Sci. USA 85:5879-5883, 1988). Such single chain antibodies are also encompassed within the term "antigen-binding fragment."

Production of antibodies and antibody fragments is well documented in the field. See, e.g., Harlow and Lane, 1988. Antibodies, A Laboratory Manual. Cold Spring Harbor, New York: Cold Spring Harbor Laboratory. For example, Jones et al., Nature 321: 522-525, 1986, which discloses replacing the CDRs of a human antibody with those from a mouse antibody. Marx, Science 229:455-456, 1985, discusses chimeric antibodies having mouse variable regions and human constant regions. Rodwell, Nature 342:99-100, 1989, discusses lower molecular weight recognition elements derived from antibody CDR information. Clackson, Br. J. Rheumatol. 3052: 36-39, 1991, discusses genetically engineered monoclonal antibodies, including Fv fragment derivatives, single chain antibodies, fusion proteins chimeric antibodies and humanized rodent antibodies. Reichman et al., Nature 332:323-327, 1988 discloses a human antibody on which rat hypervariable regions have been grafted. Verhoeyen, et al., Science 239:1534-1536, 1988, teaches grafting of a mouse antigen binding site onto a human antibody.

### Small Molecule Drugs

We also disclose drugs (*e.g*., small molecule drugs) that promote BMP protein expression or activity. In some embodiments, such small molecule drugs can be identified using the drug screening methods described herein. In preferred embodiments, the small molecule drugs of the present invention promote increased BMP activity and/or expression in a cell. In some embodiments, small molecule drugs are identified using the drug screens described below.

In some embodiments, a BMP agonist is a molecule disclosed in U.S. patent 7,482,329, or a peptidomimetic thereof.

### BMP Nucleic Acids

The Sca-1+ progenitor cells origin can be, for example, transfected with an exogenous nucleic acid that includes a heterologous nucleotide sequence, e.g., encoding BMP-2, -4, -5, - 6, and/or -7, or an agonist thereof, with or without a nucleotide sequence encoding a signal peptide, and produce the encoded product either transiently or stably, over extended periods of time. A heterologous amino acid can also be a regulatory sequence, e.g., a promoter, which causes expression, e.g., constitutive or inducible expression or upregulation, of an endogenous BMP-2, -4, -5, -6, and/or -7 sequence. An exogenous nucleic acid sequence can be introduced into a primary or secondary cell by homologous recombination as described, for example, in U.S. Patent No. 5,641,670, the contents of which are incorporated herein by reference. The transfected cells can also include DNA encoding a selectable marker that confers a selectable phenotype upon them, facilitating their identification and isolation.

In some embodiments of the methods described herein the compound that enhances BMP signaling as described herein includes, e.g., a BMP nucleic acid, e.g., a BMP-2, -4, -5, - 6, and/or -7 encoding sequence or active fragment thereof, and any of: a promoter sequence, e.g., a promoter sequence from a BMP-2, -4, -5, -6, and/or -7 gene or from another gene; an enhancer sequence, e.g., 5' untranslated region (UTR), e.g., a 5' UTR from a BMP-2, -4, -5, - 6, and/or -7 gene or from another gene, a 3' UTR, e.g., a 3' UTR from a BMP-2, -4, -5, -6, and/or -7 gene or from another gene; a polyadenylation site; an insulator sequence; or another sequence that enhances the expression of BMP-2, -4, -5, -6, and/or -7.

The nucleic acids described herein, e.g., a nucleic acid encoding a BMP-2, -4, -5, -6, and/or -7 polypeptide as described herein, can be incorporated into a gene construct. The methods described herein can use such expression vectors for in vitro transfection and expression of a BMP-2, -4, -5, -6, and/or -7 polypeptide described herein in particular cell types, e.g., stem cells, e.g., pluripotent mesenchymal stem cells. Expression constructs comprising a BMP nucleic acid sequence can be administered in any effective carrier, e.g., any formulation or composition capable of effectively delivering the component gene to cells *in vivo.* Approaches include insertion of the gene in viral vectors, including recombinant retroviruses, adenovirus, adeno-associated virus, lentivirus, poxvirus, alphavirus, and herpes simplex virus-1, or recombinant bacterial or eukaryotic plasmids. Viral vectors transfect cells directly; plasmid DNA can be delivered naked or with the help of, for example, cationic liposomes (lipofectamine) or derivatized (e.g., antibody conjugated), polylysine conjugates, gramicidin S, artificial viral envelopes or other such intracellular carriers, as well as direct injection of the gene construct or CaPO₄ precipitation carried out *in vivo.*

In some embodiments, the expression vector is a viral vector containing one or more BMP nucleic acid sequences (e.g., cDNA). Infection of cells with a viral vector has the advantage that a large proportion of the targeted cells can receive the nucleic acid. Additionally, molecules encoded within the viral vector, e.g., by a cDNA contained in the viral vector, are expressed efficiently in cells that have taken up viral vector nucleic acid.

Retrovirus vectors and adeno-associated virus vectors can be used as a recombinant gene delivery system for the transfer of exogenous genes *in vivo,* particularly into humans. These vectors provide efficient delivery of genes into cells, and the transferred nucleic acids are stably integrated into the chromosomal DNA of the host. The development of specialized cell lines (termed "packaging cells") which produce only replication-defective retroviruses has increased the utility of retroviruses for gene therapy, and defective retroviruses are characterized for use in gene transfer for gene therapy purposes (Reviewed in Hu and Pathak, Pharmacol. Rev. 52: 493-511 (2000); Young et al., J. Pathol. 208:229-318 (2006)). A replication defective retrovirus can be packaged into virions, which can be used to infect a target cell through the use of a helper virus by standard techniques. Protocols for producing recombinant retroviruses and for infecting cells *in vitro* or *in vivo* with such viruses can be found in Ausubel, et al., eds., Current Protocols in Molecular Biology, Greene Publishing Associates, (1989), Sections 9.10-9.14, and other standard laboratory manuals. Examples of suitable retroviruses include pLJ, pZIP, pWE and pEM which are known to those skilled in the art. Examples of suitable packaging virus lines for preparing both ecotropic and amphotropic retroviral systems include ΨCrip, ΨCre, Ψ2, ΨAm, pA12 and PA317 (For a review, see Miller et. al, Hum. Gene Ther. 1:5-14 (1990)). Retroviruses have been used to introduce a variety of genes into many different cell types, including epithelial cells, *in vitro* and/or *in vivo* (see for example Eglitis et al., Science 230:1395-1398 (1985); Danos and Mulligan, Proc. Natl. Acad. Sci. USA 85:6460-6464 (1988); Wilson et al., Proc. Natl. Acad. Sci. USA 85:3014-3018 (1988); Armentano et al., Proc. Natl. Acad. Sci. USA 87:6141-6145 (1990); Miller et al., Blood 76:271-8 (1990); Huber et al. Proc. Natl. Acad. Sci. USA 88:8039-8043 (1991); Ferry et al. Proc. Natl. Acad. Sci. USA 88:8377-8381 (1991); Chowdhury et al. Science 254:1802-1805 (1991); van Beusechem et al. Proc. Natl. Acad. Sci. USA 89:7640-7644 (1992); Kay et al. Human Gene Therapy 3:641-647 (1992); Dai et al. Proc. Natl. Acad. Sci. USA 89:10892-10895 (1992); Hwu et al. J. Immunol. 150:4104-4115 (1993); Cavazzana-Calvo et al., Science 288:669-672 (2000); U.S. Patent No. 4,868,116; U.S. Patent No. 4,980,286; PCT Application WO 89/07136; PCT Application WO 89/02468; PCT Application WO 89/05345; and PCT Application WO 92/07573).

Another viral gene delivery system useful in the present methods utilizes adenovirus-derived vectors. The generation of replication-deficient adenovirus was achieved through the manipulation of the genome of an adenovirus, such that it encodes and expresses a gene product of interest but is inactivated in terms of its ability to replicate in a normal lytic viral life cycle. See, for example, Berkner et al., BioTechniques 6:616 (1988); Rosenfeld et al., Science 252:431-434 (1991); and Rosenfeld et al., Cell 68:143-155 (1992). Suitable adenoviral vectors derived from the adenovirus strain Ad type 5 dl324 or other strains of adenovirus (e.g., Ad2, Ad3, or Ad7 etc.) are known to those skilled in the art. Recombinant adenoviruses can be advantageous in certain circumstances, in that they are not capable of infecting non-dividing cells and can be used to infect a wide variety of cell types, including epithelial cells (Rosenfeld et al., (1992) supra). Furthermore, the virus particle is relatively stable and amenable to purification and concentration, and as above, can be modified so as to affect the spectrum of infectivity. Additionally, introduced adenoviral DNA (and foreign DNA contained therein) is not integrated into the genome of a host cell but remains episomal, thereby avoiding potential problems that can occur as a result of insertional mutagenesis in situ, where introduced DNA becomes integrated into the host genome (e.g., retroviral DNA). Moreover, the carrying capacity of the adenoviral genome for foreign DNA is large (up to 8 kilobases (kb)) relative to other gene delivery vectors (Berkner et al., supra; Haj-Ahmand and Graham, J. Virol. 57:267 (1986). Additionally, special high-capacity adenoviral (HC-Ad) vectors have been created that can contain more than 30 kb of transgene (Kochanek et al., Hum. Gene Ther. 10:2451-9 (1999)).

Yet another viral vector system useful for delivery of nucleic acids is the adeno-associated virus (AAV). Adeno-associated virus is a naturally occurring defective virus that requires another virus, such as an adenovirus or a herpes virus, as a helper virus for efficient replication and a productive life cycle (Reviewed in McCarty et al., Annu Rev Genet 38:819-45 (2004); Daya et al., Clin. Microbiol. Rev. 21: 583-93 (2008)). It is also one of the few viruses that may integrate its DNA into non-dividing cells, and exhibits a high frequency of stable integration that can lead to long term expression (see for example Samulski et al., J. Virol. 63:3822-3828 (1989); and McLaughlin et al., J. Virol. 62:1963-1973 (1989); Flotte et al., Am. J. Respir. Cell. Mol. Biol. 7:349-356 (1992); Miller et al., Nature Genet. 36:767-773 (2004)). Vectors containing as little as 300 base pairs of AAV can be packaged and can integrate. Space for exogenous DNA is limited to about 4 kb. An AAV vector such as that described in Tratschin et al., Mol. Cell. Biol. 5:3251-3260 (1985) can be used to introduce DNA into cells. Through the use of AAV vectors, which are derived from many different serotypes, a variety of nucleic acids have been introduced into different cell types (see for example Hermonat et al., Proc. Natl. Acad. Sci. USA 81:6466-6470 (1984); Tratschin et al., Mol. Cell. Biol. 4:2072-2081 (1985); Wondisford et al., Mol. Endocrinol. 2:32-39 (1988); Tratschin et al., J. Virol. 51:611-619 (1984); and Flotte et al., J. Biol. Chem. 268:3781-3790 (1993); Summerford et al., J. Virol. 72:1438-45 (1998); Davidson et al., Proc. Natl. Acad. Sci. USA 97:3428-32 (2000); Zabner et al., J. Virol. 74:3852-8 (2000); Rabinowitz JE et al., J. Virol. 76:791-801 (2002); Davidoff et al., Mol Ther. 11:875-88 (2005); Mueller et al., Gene Ther. 15:858-63. (2008)).

In addition to viral transfer methods, such as those illustrated above, non-viral methods can also be employed to cause expression of a nucleic acid compound described herein (for a review of such methods see Niidome et al., Gene Ther. 9:1647-52 (2002)). Typically non-viral methods of gene transfer rely on the normal mechanisms used by mammalian cells for the uptake and intracellular transport of macromolecules. In some embodiments, non-viral gene delivery systems can rely on endocytic pathways for the uptake of the subject gene by the targeted cell. Exemplary gene delivery systems of this type include liposomal derived systems, poly-cationic conjugates such as polyamine and polylysine, and artificial viral envelopes. Other embodiments include plasmid injection systems such as are described in Cohen et al., Gene Ther. 7:1896-905 (2000); Tam et al., Gene Ther. 7:1867-74 (2000); Meuli et al., J. Invest. Dermatol. 116:131-135 (2001); or Fenske et al., Methods Enzymol. 346:36-71 (2002).

In some embodiments, BMPs can be expressed using naked DNA constructs and/or DNA vector based constructs.

Naked DNA constructs and the therapeutic use of such constructs are well known to those of skill in the art (see, e.g., Chiarella et al., Recent Patents Anti-Infect. Drug Disc.,3:93-101, 2008; Gray et al., Expert Opin. Biol. Ther., 8:911-922, 2008; Melman et al., Hum. Gene Ther., 17:1165-1176, 2008). Typically, naked DNA constructs include one or more therapeutic nucleic acids (e.g., DNA encoding BMP) and a promoter sequence. A naked DNA construct can be a DNA vector, commonly referred to as pDNA. Naked DNA typically do not incorporate into chromosomal DNA. Generally, naked DNA constructs do not require, or are not used in conjunction with, the presence of lipids, polymers, or viral proteins. Such constructs may also include one or more of the non-therapeutic components described herein.

DNA vectors are known in the art and typically are circular double stranded DNA molecules. DNA vectors usually range in size from three to five kilo-base pairs (e.g., including inserted therapeutic nucleic acids). Like naked DNA, DNA vectors can be used to deliver and express one or more therapeutic proteins in target cells. DNA vectors do not incorporate into chromosomal DNA.

Generally, DNA vectors include at least one promoter sequence that allows for replication in a target cell. Uptake of a DNA vector may be facilitated (e.g., improved) by combining the DNA vector with, for example, a cationic lipid, and forming a DNA complex.

In some embodiments, DNA vectors can be introduced into target cells via conventional transformation or transfection techniques. As used herein, the terms "transformation" and "transfection" are intended to refer to a variety of art-recognized techniques for introducing foreign nucleic acid (e.g., DNA) into a target cell, including calcium phosphate or calcium chloride co-precipitation, DEAE-dextran-mediated transfection, lipofection, or electroporation.

All the molecular biological techniques required to generate an expression construct described herein are standard techniques that will be appreciated by one of skill in the art. Detailed methods may also be found, e.g., Current Protocols in Molecular Biology, Ausubel et al. (eds.) Greene Publishing Associates, (1989), Sections 9.10-9.14 and other standard laboratory manuals. DNA encoding altered an altered BMP amino acid sequence (e.g., an amino acid sequence with at least 50, 60, 70, 80, 90, 95, 98, and 99% identity to a wild type BMP sequence shown herein) can be generated using, e.g., site directed mutagenesis techniques.

### Formulations

Generally, where a BMP polypeptide or nucleic acid is used, the polypeptide or nucleic acid will be from the same species as the subject, e.g., human, cat, dog, cow, pig, or sheep.

The BMP compounds described herein can be formulated in any suitable manner, e.g., in a carrier system, for use in contacting with the populations of cells. The carrier can be a colloidal system. The colloidal system can be liposome, a phospholipid bilayer vehicle. In some embodiments, the protein is encapsulated in a liposome while maintaining protein integrity. As one skilled in the art would appreciate, there are a variety of methods to prepare liposomes. (See Lichtenberg et al., Methods Biochem Anal, 33:337-462 (1988), LIPOSOME TECHNOLOGY Anselem et al., CRC Press, 1993). Liposomes can be prepared from an assortment of phospholipids varying in size and substitution, and may also contain additional components with low toxicity, such as cholesterol. The liposome can be formulated and isolated in a variety of shapes and sizes. Additionally, moieties can be attached to the surface of the liposome to further enhance the pharmacokinetic properties of the carrier. The moieties may be attached to phospholipid or cholesterol molecules, and the percentage of the moiety incorporated on the surface may be adjusted for optimal liposome stability and pharmacokinetic characteristics. One embodiment comprises a liposome with poly-ethylene glycol (PEG) added to the surface. Liposomal formulations can delay clearance and increase cellular uptake. (See Reddy, Annals of Pharmacotherapy, 34(7/8):915-923 (2000)).

The carrier can also be a polymer, e.g., a biodegradable, biocompatible polymer matrix. In some embodiments, the protein can be embedded in the polymer matrix while maintaining protein integrity. The polymer may be natural, such as polypeptides, proteins or polysaccharides, or synthetic, such as poly(I-hydroxy) acids. Examples include carriers made of, e.g., collagen, fibronectin, elastin, cellulose acetate, cellulose nitrate, polysaccharide, fibrin, gelatin, and combinations thereof. In some embodiments, the polymer is poly-lactic acid (PLA) or co-polylactic/glycolic acid (PGLA). The polymeric matrices can be prepared and isolated in a variety of forms and sizes, including microspheres and nanospheres. Polymer formulations can lead to prolonged duration of therapeutic effect. (See Reddy, Annals of Pharmacotherapy, 34(7/8):915-923 (2000)). A polymer formulation for human growth hormone (hGH) has been used in clinical trials. (See Kozarich and Rich, Chemical Biology 2:548-552 (1998)).

Examples of polymer microsphere sustained release formulations are described in PCT publication WO 99/15154 (Tracy et al.), U.S Pat. Nos. 5,674,534 and 5,716,644 (both to Zale et al.), PCT publication WO 96/40073 (Zale et al.), and PCT publication WO 00/38651 (Shah et al.). U.S. Pat. Nos. 5,674,534 and 5,716,644 and PCT publication WO 96/40073 describe a polymeric matrix containing particles of erythropoietin that are stabilized against aggregation with a salt.

### Differentiation Methods

Described herein are methods for generating mature BAT cells or cells with the characteristic of mature BAT cells by contacting a population of Sca-1+ progenitor cells with one or more BMPs. The amount (e.g., concentration or dose) of the one or more BMPs as well as the treatment time will be sufficient to increase the number of BAT cells or cells with the characteristic of mature BAT cells in a population of Sca-1+ progenitor cells. Both the amount and the treatment time can be determined by one of skill in the art using known methods. In some embodiments, the BMP is BMP-7.

In some embodiments, the amount of the one or more BMPs can include, e.g., 0.1-50 nM per BMP.

In some embodiments, the treatment time can be, e.g., from , 1-2 days, 1-3, days, 1-4 days, 1-5 days, and 1-more than 5 days.

In some embodiments, the Sca-1+ cells will be contacted by one or more BMPs alone, e.g., in the absence of any differentiation induction step. Alternatively, Sca-1+ cells will be treated using a first solution that includes one or more BMPs and a second solution that includes one or more chemical or hormone differentiation inducers and/or thiazolidinediones. Sca-1+ cells can be exposed to these first and second solutions sequentially (e.g., first followed by second or second followed by first) or simultaneously. The amount, the treatment time, and the order of use of the first and second solutions for both the first and second solutions can be readily determined by one of skill in the art using known methods.

In some embodiments, the second solution that includes one or more chemical or hormone differentiation inducers and/or thiazolidinediones will be an osteogenic, a chondrogenic, a myogenic, or a adipogenic differentiation solution. Exemplary differentiation solutions are described in the art *(*Klien et al., J. Biol. Chem., 274:34795-34802, 1999; Hauner et al., J. Clin. Invest., 84: 1663-1670, 1989). To facilitate the differentiation of the cells described herein to or towards a BAT lineage, cells can be treated using thiazolidinediones. Alternatively or in addition, the cells described herein can be treated using an adipogenic induction cocktail containing insulin, dexamethasone, triiodothyronine, isobutylmethylxanthine, and 0.125 mM indomethacin, for example, as previously described (Klien et al., J. Biol. Chem., 274:34795-34802, 1999).

In some embodiments, the differentiation treatment will be assessed by determining the presence, and or absence, of one or markers in the cell population.

In some embodiments, the methods include evaluating the level of adipogenesis in the BMP-treated cell or cell population. Adipogenesis can be evaluated by measuring one or more of, e.g., lipid accumulation (e.g., using oil red-o (ORO) staining. Oil Red O dye is used to stain neutral lipids in cells. The amount of staining is directly proportional to the amount of lipid in the cell and can be measured spectrophotometrically. The amount of lipid accumulation is determined as a parameter of differentiation. Alternatively or in addition, adipogenesis can be evaluated by measuring one or more markers of adipogenesis, e.g., selected from peroxisome proliferator activated receptor gamma (PPARK), fatty acid binding protein 4 (FABP4, aP2), and/or fatty acid synthase (FAS).

In some embodiments, the methods include evaluating the level of BAT adipogenesis in the cell or cell population. BAT differentiation can be evaluated by measuring one or more of, e.g., one or more BAT specific markers, such as uncoupling protein (UCP), cell death-inducing DFF45-like effector A (CIDEA), PPAR gamma coactivator (PGC)-1 alpha, and/or PPAR gamma coactivator (PGC)-1 beta and/or PRDM-16; BAT morphology (e.g., using visual, e.g., microscopic, inspection of the cells); or BAT thermodynamics, e.g., cytochrome oxidase activity, Na+-K+-ATPase enzyme units, or other enzymes involved in BAT thermogenesis.

In some embodiments, the methods can include evaluating the level of preadipocyte factor (pref)-1, an inhibitor of adipogenesis in a cell, wherein a decrease in the level of pref-1, e.g., relative to the level of pref-1 in a suitable untreated control cell or the same cell prior to treatment, indicates that the cell has undergone BAT differentiation.

In some embodiments, the methods include treating cells with cyclic AMP (cAMP), or an analogue thereof, such as dibutryl cAMP, or ϑ3-adrenergic agonist CL316249 to assess the ability of the cells to activate thermogenesis. Cold-induced thermogenesis *in vivo* is mediated through a signaling cascade involving the sympathetic nervous system and activation of the ϑ3-adrenergic receptor in BAT. These events result in an increase of cytoplasmic cAMP levels, which then triggers expression of genes involved in thermogenesis in mature brown adipocytes. To determine if the differentiated cells become *bona fide* brown adipocytes, the expression of thermogenic genes, such as UCP-1, in differentiated adipocytes treated with the cell-penetrant cAMP analogue dibutyryl cAMP (Sigma) or ϑ3-adrenergic agonist CL316249 (Sigma) can be measured. These methods include assessing (e.g., measuring) the expression of one or more genes involved in thermogenesis in mature brown adipocytes. Exemplary genes include, but are not limited to, UCP-1, CIDEA, PGC-1, PRDM16, and genes involved in mitochondrial biogenesis and function. Cells that show expression of one or more of these genes are identified as mature BAT cells and/or cells with characteristics of a mature brown adipocyte.

In some embodiments, the methods include evaluating WAT differentiation, e.g., evaluating a WAT specific marker, such as one or more of resistin, TCF21, leptin and/or nuclear receptor interacting protein 1 (RIP140), and/or WAT morphology.

WAT and BAT can be distinguished by routine techniques, e.g., morphologic changes specific to WAT or BAT, or evaluation of WAT-specific or BAT-specific markers. For example, BAT cells can be identified by expression of uncoupling protein (UCP), e.g., UCP-1.

### Methods of Treatment

The methods and compositions described herein are useful for the treatment of obesity and weight-related disorders. Generally, the methods include administering an effective amount of BMP-treated cells to a subject in need thereof, including a subject that has been diagnosed to be in need of such treatment.

### Subject Selection

In some embodiments, the methods described herein include identifying a subject in need of treatment (e.g., an overweight or obese subject, e.g., with a body mass index (BMI) of 25-29 or 30 or above or a subject with a weight related disorder) and administering to the subject an effective amount of BMP-treated cells. A subject in need of treatment with the methods described herein can be selected based on the subject's body weight or body mass index. In some embodiments, the methods include evaluating the subject for one or more of: weight, adipose tissue stores, adipose tissue morphology, insulin levels, insulin metabolism, glucose levels, thermogenic capacity, and cold sensitivity. In some embodiments, subject selection can include assessing the amount or activity of BAT in the subject and recording these observations.

The evaluation can be performed before, during, and/or after the administration of the compound. For example, the evaluation can be performed at least 1 day, 2 days, 4, 7, 14, 21, 30 or more days before and/or after the administration.

In some embodiments, Sca-1+ progenitor cells can be obtained from a subject selected for treatment.

### Cell Therapy

Methods described herein can include implanting a population of BMP-treated Sca-1+ cells, e.g., as described herein, into a subject to be treated, wherein said population of BMP-treated stem cells, or their progeny (i.e., daughter cells), undergo brown adipogenesis. Once implanted, the Sca-1+ cells will generally undergo adipogenesis, generating BAT in the subject.

These cell therapy methods are useful, e.g., for the treatment of obesity and insulin resistance in a subject, or for treating a disease associated with a lack of mitochondria, e.g., diabetes, cancer, neurodegeneration, and aging.

Methods for implanting the populations of BMP-treated Sca-1+ cells (e.g., Sca-1+ cells that have been treated using the differentiation methods described herein) are known in the art, e.g., using a delivery system configured to allow the introduction of cells into a subject. In general, the delivery system can include a reservoir containing a population of cells including BMP-treated Sca-1+ cells, and a needle in fluid communication with the reservoir. Typically, the population of BMP-treated Sca-1+ cells will be in a pharmaceutically acceptable carrier, with or without a scaffold, matrix, or other implantable device to which the cells can attach (examples include carriers made of, e.g., collagen, fibronectin, elastin, cellulose acetate, cellulose nitrate, polysaccharide, fibrin, gelatin, and combinations thereof). Such delivery systems are also within the scope of the invention. Generally, such delivery systems are maintained in a sterile manner. Various routes of administration and various sites (e.g., renal sub capsular, subcutaneous, central nervous system (including intrathecal), intravascular, intrahepatic, intrasplanchnic, intraperitoneal (including intraomental), intramuscularly implantation) can be used. Generally, the cells will be implanted into the subject subcutaneously. In some embodiments, the population of BMP-treated Sca-1+ cells that is implanted includes at least 10⁷,10⁸, 10⁹, or more cells.

Where non immunologically compatible cells are used, an immunosuppressive compound e.g., a drug or antibody, can be administered to the recipient subject at a dosage sufficient to achieve inhibition of rejection of the cells. Dosage ranges for immunosuppressive drugs are known in the art. See, e.g., Freed et al., N. Engl. J. Med. 327:1549 (1992); Spencer et al., N. Engl. J. Med. 327:1541 (1992); Widner et al., N. Engl. J. Med. 327:1556 (1992)). Dosage values may vary according to factors such as the disease state, age, sex, and weight of the individual.

In some embodiments, the methods include contacting, administering or expressing one or more other compounds in addition to the BMP-treated cells, e.g., peroxisome proliferator-activated receptor gamma (PPAR(), Retinoid X receptor, alpha (RxR∀), insulin, T3, a thiazolidinedione (TZD), retinoic acid, another BMP protein (e.g., BMP-1 or BMP-3), vitamin A, retinoic acid, insulin, glucocorticoid or agonist thereof, Wingless-type (Wnt), e.g., Wnt-1, Insulin-like Growth Factor-1 (IGF-1), or other growth factor, e.g., Epidermal growth factor (EGF), Fibroblast growth factor (FGF), Transforming growth factor (TGF)-I, TGF-ϑ, Tumor necrosis factor alpha (TNFI), Macrophage colony stimulating factor (MCSF), Vascular endothelial growth factor (VEGF) and/or Platelet-derived growth factor (PDGF). In other embodiments, the compound can be a BMP-2, -4, -5, -6, and/or -7 protein as described herein or a portion thereof linked with a heterologous polypeptide sequence, e.g., a second BMP protein, to form a chimeric molecule or fusion protein. In some embodiments, the methods include administering the compound in combination with a second treatment, e.g., a second treatment for obesity or a related disorder such as diabetes. For example, the second treatment can be insulin, orlistat, phendimetrazine, and/or phentermine.

### In Vivo Therapy

In some embodiments, the present disclosure provides in vivo treatment methods. Such methods include selecting a subject as described above, and administering to the subject one or more compounds that enhance BMP signaling to the subject, wherein each of the compounds specifically target Sca-1+ cells in the subject. Compounds that can be used in such methods include, for example, BMPs and BMP agonists that specifically target Sca-1+ cells. Such compounds can bind specifically to Sca-1+ and/or CD29 receptors on the surface of cells.

### Effective Dose

Toxicity and therapeutic efficacy of the compounds and pharmaceutical compositions described herein can be determined by standard pharmaceutical procedures, using either cells in culture or experimental animals to determine the LD₅₀ (the dose lethal to 50% of the population) and the ED₅₀ (the dose therapeutically effective in 50% of the population). The dose ratio between toxic and therapeutic effects is the therapeutic index and can be expressed as the ratio LD₅₀/ED₅₀. Polypeptides or other compounds that exhibit large therapeutic indices are preferred.

Data obtained from cell culture assays and further animal studies can be used in formulating a range of dosage for use in humans. The dosage of such compounds lies preferably within a range of circulating concentrations that include the ED₅₀ with little or no toxicity, and with little or no adverse effect on a human's ability to hear. The dosage may vary within this range depending upon the dosage form employed and the route of administration utilized. For any compound used in the methods described herein, the therapeutically effective dose can be estimated initially from cell culture assays. A dose can be formulated in animal models to achieve a circulating plasma concentration range that includes the IC₅₀ (that is, the concentration of the test compound which achieves a half-maximal inhibition of symptoms) as determined in cell culture. Such information can be used to more accurately determine useful doses in humans. Exemplary dosage amounts of a differentiation agent are at least from about 0.01 to 3000 mg per day, *e.g.,* at least about 0.00001, 0.0001, 0.001, 0.01., 0.1, 1, 2, 5, 10, 25, 50, 100, 200, 500, 1000, 2000, or 3000 mg per kg per day, or more.

The formulations and routes of administration can be tailored to the disease or disorder being treated, and for the specific human being treated. A subject can receive a dose of the agent once or twice or more daily for one week, one month, six months, one year, or more. The treatment can continue indefinitely, such as throughout the lifetime of the human. Treatment can be administered at regular or irregular intervals (once every other day or twice per week), and the dosage and timing of the administration can be adjusted throughout the course of the treatment. The dosage can remain constant over the course of the treatment regimen, or it can be decreased or increased over the course of the treatment.

Generally the dosage facilitates an intended purpose for both prophylaxis and treatment without undesirable side effects, such as toxicity, irritation or allergic response. Although individual needs may vary, the determination of optimal ranges for effective amounts of formulations is within the skill of the art. Human doses can readily be extrapolated from animal studies (Katocs et al., Chapter 27 In: Remington's Pharmaceutical Sciences, 18th Ed., Gennaro, ed., Mack Publishing Co., Easton, Pa., 1990). Generally, the dosage required to provide an effective amount of a formulation, which can be adjusted by one skilled in the art, will vary depending on several factors, including the age, health, physical condition, weight, type and extent of the disease or disorder of the recipient, frequency of treatment, the nature of concurrent therapy, if required, and the nature and scope of the desired effect(s) (Nies et al., Chapter 3, In: Goodman & Gilman's "The Pharmacological Basis of Therapeutics", 9th Ed., Hardman et al., eds., McGraw-Hill, New York, N.Y, 1996).

### Assessment/Validation of Treatment

In some embodiments, the methods described herein can include assessing the amount or activity of BAT in the subject following treatment and recording these observations. These post-treatment observations can be compared to the observations made during subject selection. In some embodiments, the subject will have increased BAT levels and/or activity. In some embodiments, the subject will show reduced symptoms.

In some embodiments, assessment can include determining the subject's weight or BMI before and/or after treatment, and comparing the subject's weight or BMI before treatment to the weight or BMI after treatment. An indication of success would be observation of a decrease in weight or BMI. In some embodiments, the treatment is administered one or more additional times until a target weight or BMI is achieved. Alternatively, measurements of girth can be used, e.g., waist, chest, hip, thigh, or arm circumference.

These assessments can be used to determine the future course of treatment for the subject. For example, treatment may be continued without change, continued with change (e.g., additional treatment or more aggressive treatment), or treatment can be stopped.

In some embodiments, the methods include one or more additional rounds of implantation of BMP activated cells, e.g., to increase brown adipogenesis, e.g., to maintain or further reduce obesity in the subject.

### Screening Methods

In some embodiments the methods described herein include screening methods for identifying BAT progenitor cells. These methods include obtaining Sca-1+ progenitor cells. In some embodiments, these cells will be characterized, e.g., by assessing cell surface marker expression, RNA and DNA profiling, and proteomics. Alternatively or in addition, the cells can be treated using the differentiation methods described herein. In some embodiments, the Sca-1+ cells can be treated using BMP-7 alone or in combination with one or more differentiation induction cocktails. The cells can then be evaluated to confirm that the population of cells includes one or more mature BAT cells or cells with characteristics of BAT cells. These mature BAT cells or cells with characteristics of BAT cells can then be isolated and characterized, e.g., by assessing cell surface marker expression, RNA and DNA profiling, and proteomics. The observations made will be recorded and compared to observations made using similarly treated Sca-1+ cells. In some embodiments, the observations made will be compared to the observations made using the untreated progenitor cells to determine which markers are present before and after differentiation. Recurrent observations will then be determined and used to build a profile for cells that are capable of undergoing differentiation to or towards a BAT cell lineage. This profile can then be used to select BAT progenitor cells from a heterogeneous cell population.

### Kits

The disclosure also features kits. In some embodiments, the kits can include (1) one or more Sca-1+ progenitor cells; (2) one or more BMPs capable of promoting the differentiation of the one or more Sca-1+ progenitor cells to or towards a mature BAT cell lineage; (3) a device for administering the cells to a subject; (4) instructions for administration; and optionally (5) one or more differentiation induction cocktails.

In some embodiments, the kits can include (1) one or more BMP-treated Sca-1+ cells; (2) a device for administering the cells to a subject; and (3) instructions for administration. Embodiments in which two or more, including all, of the components are found in the same container are included.

When a kit is supplied, the different components of the compositions included can be packaged in separate containers and admixed immediately before use. Such packaging of the components separately can permit long term storage without loosing the active components' functions. When more than one bioactive agent is included in a particular kit, the bioactive agents may be (1) packaged separately and admixed separately with appropriate (similar of different, but compatible) adjuvants or excipients immediately before use, (2) packaged together and admixed together immediately before use, or (3) packaged separately and admixed together immediately before use. If the chosen compounds will remain stable after admixing, the compounds may be admixed at a time before use other than immediately before use, including, for example, minutes, hours, days, months, years, and at the time of manufacture.

The compositions included in particular kits of the present invention can be supplied in containers of any sort such that the life of the different components are optimally preserved and are not adsorbed or altered by the materials of the container. Suitable materials for these containers may include, for example, glass, organic polymers (e.g., polycarbonate and polystyrene), ceramic, metal (e.g., aluminum), an alloy, or any other material typically employed to hold similar reagents. Exemplary containers may include, without limitation, test tubes, vials, flasks, bottles, syringes, and the like.

As stated above, the kits can also be supplied with instructional materials. These instructions may be printed and/or may be supplied, without limitation, as an electronic-readable medium, such as a floppy disc, a CD-ROM, a DVD, a Zip disc, a video cassette, an audiotape, and a flash memory device. Alternatively, instructions may be published on a internet web site or may be distributed to the user as an electronic mail.

The kits also include kits for the treatment or prevention of weight-related disorders and obesity.

The invention is further described in the following examples, which do not limit the scope of the invention described in the claims.

### EXAMPLES

### Example 1: Bone Morphogenic Protein Treatment Promotes BAT Differentiation in the Absence of Chemical and/or Hormonal Differentiation Inducers

The effect of treating wild-type brown preadipocytes and 3T3-L1 white preadipocytes with bone morphogenic protein (BMP) -2, -3, -4, -5, -6, and -7 was analyzed according to the following methods.

Sub confluent cells were cultured in Dulbecco's Modified Eagles Medium (DMEM) supplemented with 10% fetal bovine serum and either BMP-2, BMP-3, BMP-4, BMP-5, BMP-6, or BMP-7. Control cell media was devoid of BMP. Cells were not exposed to chemical or hormonal differentiation inducers or thiazolidinediones. Cells were cultured for eight days. Cells were then collected for Oil Red O staining and RNA extraction.

Lipid accumulation was analyzed using Oil Red O staining. Briefly, cells were stained for two hours at room temperature with filtered Oil Red O solution (0.5% Oil Red O (Sigma-Aldrich) in isopropyl alcohol).

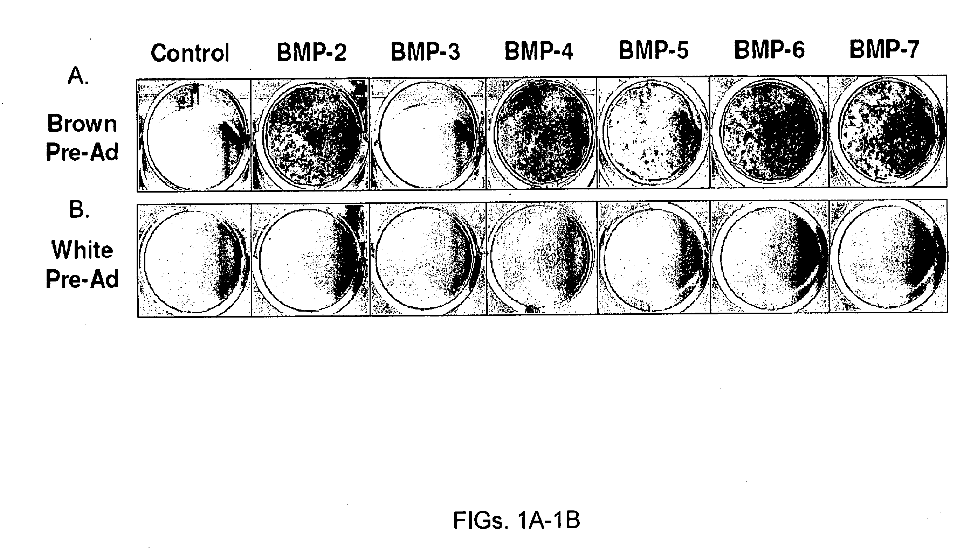

As shown in FIG. 1A, BMP-2, BMP-4, BMP-6, and BMP-7 caused a marked increase in lipid accumulation in wild-type brown preadipocytes compared to the control cell population, as indicated by the darker appearance of these cells due to increased Oil Red O accumulation. In contrast, as shown in FIG. 1B, an increase in lipid accumulation was not observed in 3T3-L1 white preadipocytes.

These data suggest that various BMPs (BMP-2, BMP-4, BMP-6, and BMP-7) are capable of promoting increased lipid accumulation in brown preadipocytes even in growth media devoid of chemical or hormonal differentiation inducers or thiazolidinediones. As lipid accumulation is consistent with the differentiation of these cells towards a BAT phenotype, these results indicate that BMP-2, BMP-4, BMP-6, and BMP-7 are capable of promoting the differentiation of BAT progenitor cells to or towards a BAT lineage.

Uncoupling protein- (UCP-1) and cell death-inducing DFF45-like effector A (CIDEA) are specific markers commonly used to confirm BAT differentiation (Zhou et al., Nat. Genet., 35: 49-56, 2003). To analyze whether BMP-2, BMP-4, BMP-6, and BMP-7 promote differentiation of the brown preadipocytes to mature BAT cells, quantitative RT-PCR was performed, using cells prepared as described above, to assess the expression of UCP-1 and CIDEA.

Wild-type brown preadipocytes were cultured in the presence of various BMPs for eight days. Cells were then harvested and RNA was isolated using QIAzol lysis reagent (Qiagen, Valencia, CA). The sense and antisense primers used to amplify UCP-1 were purchased from SuperArray (Catalogue No. PPM05164A).

The sense and antisense primers used to amplify CIDEA were, respectively:
5'- ATCACAACTGGCCTGGTTACG-3' SEQ ID NO:6; and
5'-TACTACCCGGTGTCCATTTCT-3' SEQ ID NO:7.

cDNA was prepared from 1 µg of RNA using the Advantage RT-PCR kit, according to manufacturer's instructions (BD Biosciences, Palo Alto, CA), and diluted to a final volume of 250 µl. 5 µl of diluted cDNA was used in a 20 µl PCR reaction with SYBR Green Master Mix (Applied Biosystems, Foster City, CA). Primers were used at a concentration of 300 nM each. PCR reactions were run in duplicate for each sample and quantitated using the ABI Prism 7000 Sequence Detection System (Applied Biosystems).

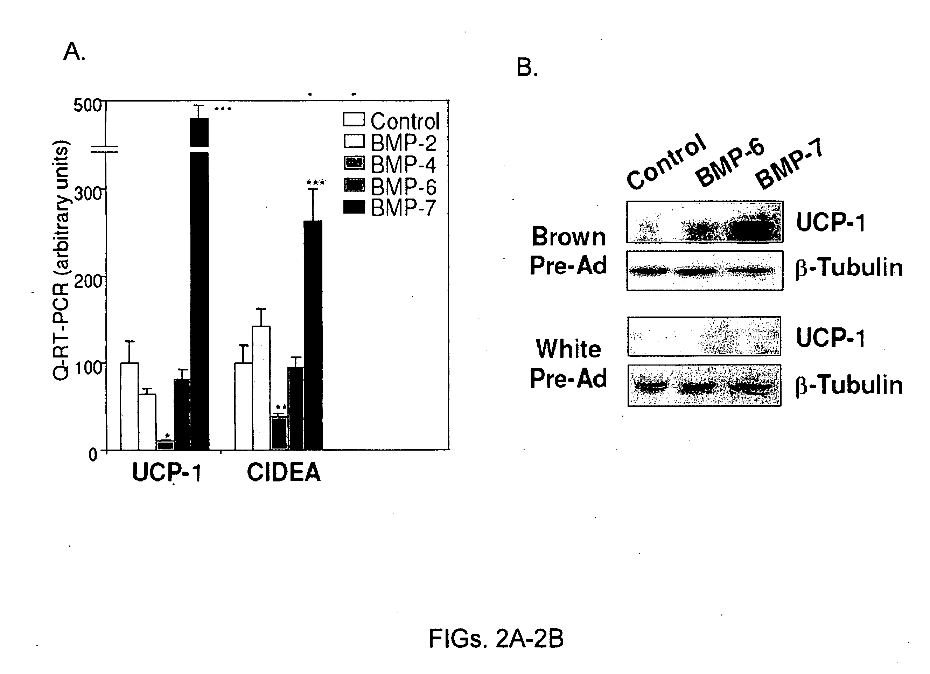

As shown in FIG. 2A, BMP-7 treated cells exhibited a marked increase in UCP-1 and CIDEA expression compared to the control cell population. An increase in CIDEA expression was also observed in BMP-2 treated cells.

These results suggest that BMP-2 and BMP-7 promote wild-type brown preadipocyte differentiation to BAT.

The above observations were also confirmed using Western Blotting. Briefly, sub-confluent brown and white preadipocytes were cultured in control media or media supplemented with 3.3nM of BMP-6 or BMP-7 for 13 days.

Protein extracts obtained from BMP-6, BMP-7, and control treated brown and white preadipocytes were resolved using SDS-PAGE and analyzed using Western blotting. Membranes were probed first using an antibody specific against UCP-1 (Santa Cruz, CA) before being stripped and re-probed using an antibody specific against ϑ-tubulin as a loading control.

As shown in FIG. 2B, UCP-1 was specifically unregulated in the brown cell population exposed to BMP-7. UCP-1 upregulation was not observed in the white cell population exposed to BMP-7 or the BMP-6 or control treated brown and white cells.

In addition to the observations described above, BMP-7 also promoted increased mitochondrial biogenesis.

These data suggest that BMP-7 is an important brown adipogenic factor and thermogenic modulator.

### Example 2: Isolation and Characterization of Stem Cell Antigen-1 Positive Brown Adipocyte Progenitor Cells

Candidate brown fat progenitor cells were isolated from adult mouse skeletal muscle. The ability of these isolated cells to differentiate to BAT cells was then investigated by exposing the cells to BMP-7.

A stem cell antigen-1 positive (Sca-1+) and CD45/Mac-1 negative (Sca-1+/CD45-/Mac-1-) population of non-myogenic progenitor cells from the myofiber-associated intertestinal cells of mouse limb was purified by fluorescence activated cell sorting (FACS). Briefly, animals were sacrificed with carbon dioxide followed by cervical dislocation. Muscle tissue was dissected from both fore and hind limbs and collected in 15ml of Digestion Buffer I (DMEM, 0.2% Collagenase Type II, Invitrogen # 17101-015). Samples were digested for 90 minutes with gentle agitation in a 37°C water bath. Digestion was stopped by addition of 3ml of fetal bovine serum (FBS). Tissue pieces were separated and washed twice with F10+20% FBS medium and one time with phosphate buffered saline (PBS) without destroying the tissue pieces. Tissue was minced in PBS to obtain intact muscle fibers.

The muscle fibers were then washed by sedimentation three to four times to remove interstitial cells. After the final wash, the supernatant was removed to a final volume of 3-5ml, followed by the addition of 5-6ml of Digestion Buffer II (F10, 0.0125 % Collagenase Type II, 0.05% Dispase, Invitrogen # 17105-041).

Samples were then incubated at 37°C for 30min with gentle agitation. Digestion was then stopped by adding of 1.5ml of FBS to the solution. Myofiber associated cells present in the sample were liberated by pipetting the sample up and down about four times. Samples were then centrifuged at low speed for one minute to remove undigested tissue pieces. The supernatant was then filtered through a 70µm cell strainer. Cells were collected by centrifugation and re-suspended in a small volume of sorting medium (2% FBS in Hanks buffered salt solution - HBSS) for antibody staining.

After one wash step in sorting medium, cells were filtered through a gauge net. After addition of viability selection markers, propidium iodide (used to detect dead cells) and calcein blue (used to detect live cells), cells were subjected to fluorescence activated cell sorting (FACS) with negative selection for PE (expression of Mac-1 and CD45) and positive selection for APC (Sca-1 expression). All antibodies were added at a dilution of 1:200 to the cell suspension and incubated for 20min on ice. Antibodies used included antibodies against Sca-1 (APC-labeled; eBioscience # 17-5981-82), Mac-1 (PE-labeled; eBioscience # 12-0112-82) and CD45 (PE-labeled; eBioscience # 12-0451-82).

Cells were then re-suspended in growth medium (F10 with 10% FBS and glutamine) and plated at a density of 25.000 cells per well on laminin/collagen-coated 24-well cell culture plate. During the growth period, cells were supplemented with daily additions of 5ng/ml of basic fibroblast growth factor (bFGF, Sigma-Aldrich # F0291).

Sca-1+/CD45-/Mac-1- progenitor cells were then exposed DMEM supplemented with 10% fetal bovine serum and 3.3nM BMP-7 for three days. Control cells were exposed to DMEM supplemented with 10% fetal bovine serum without BMP-7. Cells were then exposed to adipocyte induction medium for four days. Specifically, adipocyte differentiation was induced by treating confluent cells for 48 hours in medium supplemented with 20 nM insulin and 1 nM triiodothyronine (T3), 0.5 TM isobutylmethylxanthine (IBMX), 5 mM dexamethasone, and 0.125 mM indomethacin. After this induction period (day 2), cells were placed back to growth medium supplemented with insulin and T3, which was then changed every second day Cells were then fixed for Oil Red O staining and RNA was isolated for quantitative RT-PCR.

Oil Red O staining was performed as described in Example 1.

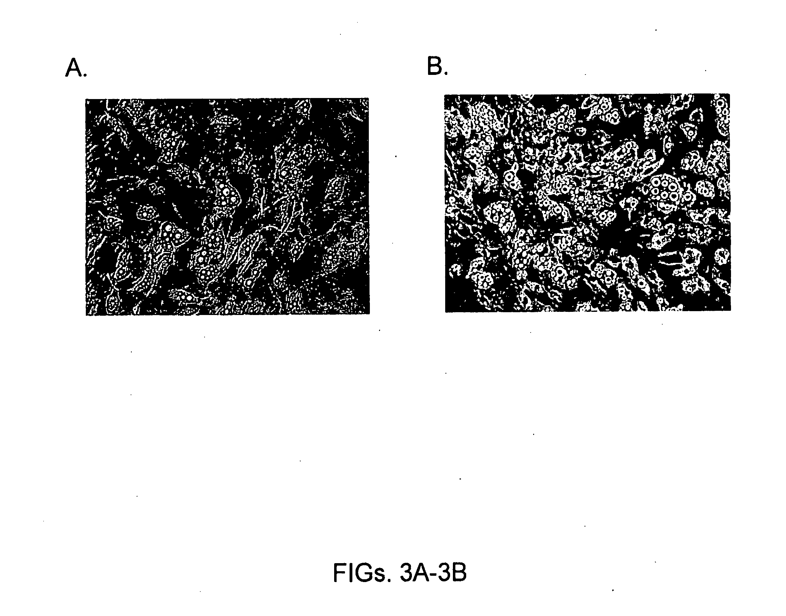

As shown in FIG. 3A and 3B, BMP-7 treated Sca-1+/CD45-/Mac-1- exhibited a marked increase in lipid accumulation compared to the control cell population, respectively.

Quantitative RT-PCR was then performed to determine the expression levels of both adipogenic and adipogenic markers that are specific to brown adipocytes. The adipogenic markers analyzed were peroxisome proliferator activated receptor gamma (PPARK), fatty acid binding protein 4 (FABA4, aP2), and fatty acid synthase (FAS). The adipogenic markers specific to brown adipocytes were UCP-1, CIDEA, PPARK coactivator (PGC)-II, and PGC-1ϑ.

Quantitative RT-PCR was performed as described in Example 1. The sense and antisense markers used to amplify PPARK were, respectively:
5'-TCAGCTCTGTGGACCTCTCC-3' SEQ ID NO:8; and
5'-ACCCTTGCATCCTTCACAAG-3' SEQ ID NO:9.

The sense and antisense markers used to amplify FABA, aP2 were, respectively:
5'-GATGCCTTTGTGGGAACCT-3' SEQ ID NO:10; and
5'-CTGTCGTCTGCGGTGATTT-3' SEQ ID NO:11.

The sense and antisense markers used to amplify FAS were, respectively:
5'- GAGGACACTCAAGTGGCTGA-3' SEQ ID NO:12; and
5'- GTGAGGTTGCTGTCGTCTGT-3' SEQ ID NO:13.

The sense and antisense markers used to amplify PGC-1I were, respectively:
5'-GTCAACAGCAAAAGCCACAA-3' SEQ ID NO:14; and
5'-TCTGGGGTCAGAGGAAGAGA-3' SEQ ID NO:15.

The sense and antisense markers used to amplify PGC-1ϑ were, respectively:
5'-CTTGCTTTTCCCAGATGAGG-3' SEQ ID NO:16; and
5'-CCCTGTCCGTGAGGAACG-3' SEQ ID NO:17.

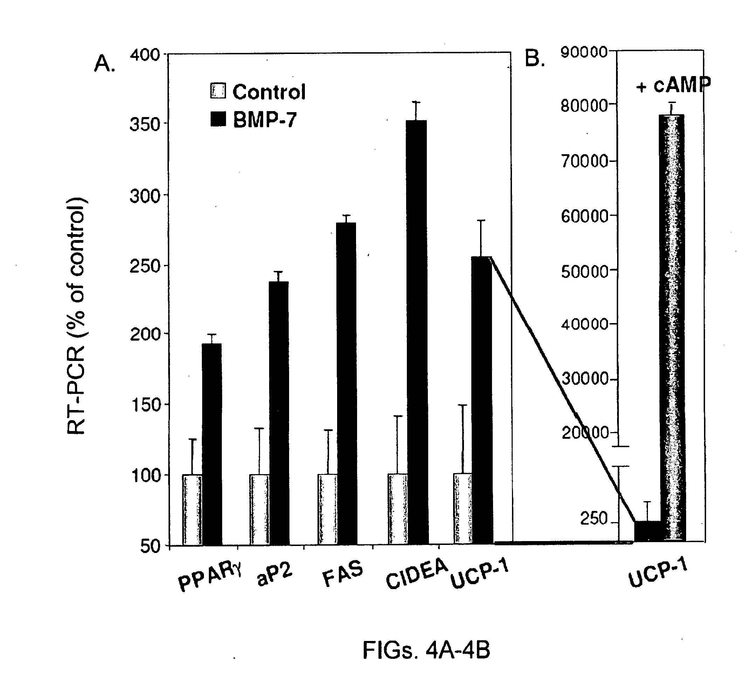

As shown in FIG. 4A, BMP-7 treatment resulted in a marked increase in both the adipogenic markers (PPARK, FABP4, aP2, and FAS) and the brown-fat selective markers (UCP-1 and CIDEA). The lowest induction was observed for PPARK, which presented a 2-fold increase over the control. All other markers presented a greater than 2-fold increase over the control. The greatest increase was observed for CIDEA, which presented a 3.5-fold increase over the control.

These observations suggest that BMP-7 treatment promotes the differentiation of Sca-1+/CD45-/Mac-1- progenitor cells to genuine BAT cells.

To further corroborate that the BMP-7 treated Sca-1+/CD45-/Mac-1- cells had differentiated to mature brown adipocytes capable of thermogenesis, the BMP-7 treated Sca-1+/CD45-/Mac-1- cells and untreated control cells were exposed to either the cyclic AMP (cAMP) analogue dibutryl cAMP (Sigma) or the ϑ3-adrenergic agonist CL316249 (Sigma). Each of these compounds mimic the induction of cold-induced thermogenesis and thus trigger the expression of genes involved in thermogenesis, which occurs exclusively in mature brown adipocytes. Either of these compounds are commonly used to functionally characterize brown adipocytes and distinguish brown adipocytes from white adipocytes.

Following treatment UCP-1 expression was analyzed using quantitative RT-PCR, as described above.

As shown in FIG. 4B, the BMP-7 treated Sca-1+/CD45-/Mac-1- cell population showed about a 300-fold increase in UCP-1 expression following cAMP compared to the control cell population. This observation suggests that Sca-1+/CD45-/Mac-1- progenitor cells exposed to BMP-7 differentiate to genuine brown adipocytes with a complete capacity to respond to catecholamine stimulation to turn on the thermogenic program.

Together, these data strongly suggest that BMP-7 can induce the differentiation of Sca-1+ progenitor cells, particularly Sca-1+/CD45-/Mac-1- progenitor cells, into *bona fide* brown adipocytes.

### Example 3: Identification of Novel Brown Adipocyte Progenitor Cells

The results presented herein demonstrate that the BMPs are able to specify brown versus white adipose fate in the Sca-1+ mesenchymal progenitor population. Sca-1 expression marks a heterogeneous precursor pool. To identify sources of Sca-1+ cells in addition to those described in Examples 1 and 2, and markers in addition to Sca-1 on the surface of brown adipocyte progenitor cells that can be used in the identification or purification of these cells, Sca-1+ progenitors were isolated from various sources, including muscle, and adipose tissue, including interscapular BAT, subcutaneous white adipose tissue, and visceral white adipose tissue, by FACS. Isolated Sca-1+ cells were then exposed to BMP7 to asses the capability of the cells to undergo differentiation to a mature brown adipocyte. Cell surface markers were then determined for cells capable of differentiating to brown adipocytes. These additional BAT progenitor cell specific cell surface markers can be used in the identification of brown adipocyte progenitor cells.

More specifically, non-hematopoetic Sca-1+ progenitor cells were isolated from skeletal muscle, bone marrow, and adipose tissue and depots, including interscapular BAT, subcutaneous white adipose tissue, and visceral white adipose tissue, as follows:

### Skeletal Muscle Derived Sca-1+ Cells

Sca-1+ cells were isolated and cultured according to the methods used in Examples 1 and 2. Specifically, animals were sacrificed with carbon dioxide followed by cervical dislocation. Muscle tissue was dissected from both fore and hind limbs and collected in 15ml of Digestion Buffer I (DMEM, 0.2% Collagenase Type II, Invitrogen # 17101-015).

After digestion for 90 minutes with gentle agitation in a 37°C water bath, digestion was stopped by addition of 3ml of fetal bovine serum (FBS). Tissue pieces were separated and washed twice with F10+20% FBS medium and one time with phosphate buffered saline (PBS) without destroying the tissue pieces. Tissue was minced in PBS to obtain intact muscle fibers.

The muscle fibers were then washed by sedimentation for three to four times to remove interstitial cells. After the final wash, the supernatant was removed to a final volume of 3-5ml followed by the addition of 5-6ml of Digestion Buffer II (F10, 0.0125 % Collagenase Type II, 0.05% Dispase, Invitrogen # 17105-041).

The samples were then incubated at 37°C for 30 minutes with gentle agitation and digestion was halted by addition of 1.5ml of FBS. After aspirating the digested sample up and down for four times to liberate myofiber associated cells, it was centrifuged at low speed for one minute to remove undigested tissue pieces. The supernatant was then filtered through a 70µm cell strainer. Cells were collected by centrifugation and re-suspended in a small volume of sorting medium (2% FBS in Hanks buffered salt solution- HBSS) for antibody staining.

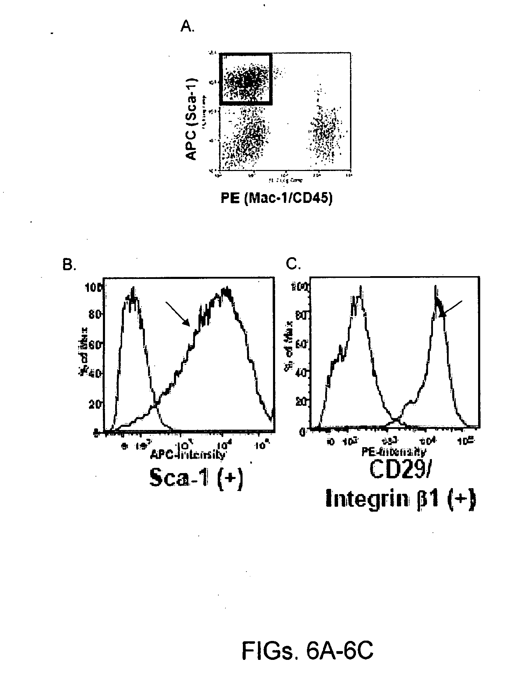

All antibodies were added at a dilution of 1:200 to the cell suspension and incubated for 20min on ice. Antibodies directed against Sca-1 (APC-labeled; eBioscience # 17-5981-82), Mac-1 (PE-labeled; eBioscience # 12-0112-82) and CD45 (PE-labeled; eBioscience # 12-0451-82). After one wash step in sorting medium, cells were filtered through a gauge net. After addition of viability selection markers, propidium iodide (dead cells) and calcein blue (life cells), the cells were subjected to fluorescence activated cell sorting (FACS) with negative selection for PE (expression of Mac-1 and CD45) and positive selection for APC (Sca-1 expression) (see FIG. 6A).

Cells were then re-suspended in growth medium (published in: Calcif Tissue Int, 2008 Jan;82(1):44-56). Growth medium consists of 60% Dulbecco's Modified Eagle Medium (D-MEM) with low glucose (Invitrogen, 11885-084) and 40% MCDB201-medium (Sigma-Aldrich, M6770). Growth medium was supplemented with 2% fetal bovine serum, penicillin/streptomycin (Invitrogen, 15140-155), 1nM dexamethsone (Sigma-Aldrich, D-4902), 0.1mM L-Ascorbic acid 2-phosphate (Sigma-Aldrich, A8960), 1x insulin transferrin selenium (ITS) mix (Sigma-Aldrich, I3146), and 1x Linoleic acid-Albumin (Sigma-Aldrich Aldrich, L9530). Cells were plated at a density of 25.000 - 50.000 cells per well on matrigel-coated 24-well cell culture plates. The growth medium was also supplemented with the following growth factors: 10ng/ml EGF (PeproTech, 315-09), 10ng/ml LIF (ESGRO from Millipore, ESG1107), 10ng/ml PDGF-BB (PeproTech, 315-18) and 5ng/ml bFGF (Sigma-Aldrich, F0291). Growth factors were added fresh to the medium prior to every medium change every other day. During the growth period, cells were supplemented with daily additions of 5ng/ml of basic fibroblast growth factor (bFGF, Sigma-Aldrich # F0291).

These isolated cells were tested for their ability to generate brown adipocytes following treatment with BMP7, as follows. Briefly, isolated Sca-1+ cells were contacted with BMP7 in the presence of absence of adipogenic differentiation media, as previously described (Klein et al., J. Biol. Chem., 274:34795-34802, 1999; Hauner et al., J. Clin. Invest., 84:1663-1670,1989). More specifically, Cells were harvested at 90-95% confluence, or after seven days of expansion after sorting. The pre-treatment was started on the following day by addition of 3.3nM BMP-7 to growth medium containing all growth factors. Additionally, bFGF was supplemented daily as before.

After 72hrs, adipogenic differentiation was induced for 48hrs. For the adipogenic induction cocktail, growth medium without growth factors was used. Instead, the following compounds were added: 5µg/µl Insulin (Roche, 11376497001), 50µM Indomethacin (Sigma-Aldrich, I7378), 0.5µM IBMX (Sigma-Aldrich, I5879), 1µM Dexamethasone (Sigma-Aldrich, D-4902), and 1nM T3 (Sigma-Aldrich, T6397). After two days of adipogenic induction, the medium was replaced with medium containing 5µg/µl Insulin and 1nM T3 only for up to seven additional days. Adipogenesis was subsequently analyzed using Oil Red O staining and quantitative RT-PCR.

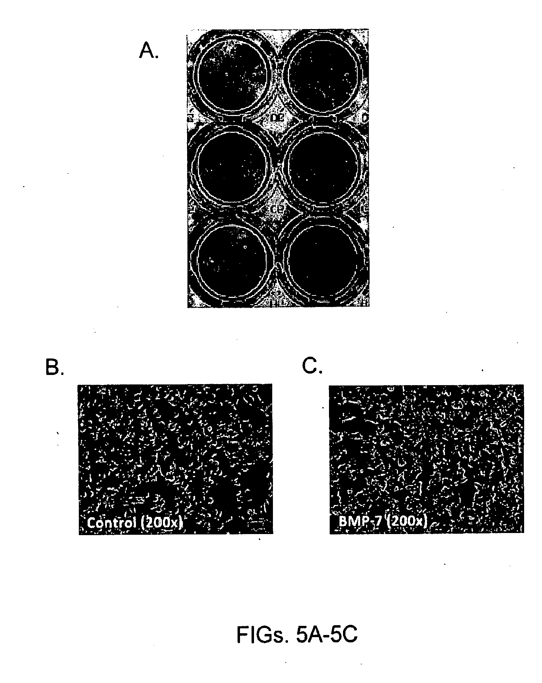

As shown in FIGs. 5A-5C, consistent with the data presented in FIG. 1, BMP7 treatment of Sca-1+ cells isolated from mouse hind limb skeletal muscle promoted a marked increase in lipid accumulation. Cell surface marker profiling of these skeletal muscle derived cells was performed prior to BMP7 treatment. As shown in FIG. 6A, these isolated skeletal muscle cells were Sca-1 positive, CD45 negative, and Mac-1 negative. As shown in FIGs. 6B-6F, these Sca-1+ BAT progenitor cells were also positive for the cell surface markers CD29/integrin ϑ1 and CD34. In contrast, the cells were negative for the cell surface markers CD31/PECAM-1 and CD117/C-kit.

These observations confirm that Sca-1+ BAT progenitor cells can be isolated from skeletal muscle. These observations also support that such cells can be identified and/or purified by positive identification of the cell surface marker Sca-1 either alone or in combination with one or more of CD29 and CD34. Such cells can be further identified by their lack of CD45, Mac-1, CD31, and CD117 cell surface expression.

To further confirm the ability of these Sca-1+ cells to differentiate to genuine brown adipocytes Quantitative RT-PCR was performed as described in Examples 1 and 2 to analyze the expression levels of adipogenic and brown adipocytes specific adipogenic markers in these skeletal muscle-derived Sca-1+ cells. In addition to those markers described in Examples 1 and 2, the expression levels of PRDM-16, ATPase, Pref-1, BMPR-11A, BMPR-1B, BMPR-2, and ACVR-1 were also detected. As described above, UCP-1 is a highly reliable marker of BAT. Similarly, PRDM-16 is a brown adipocytes specific adipogenic marker and UCP-1 is a general marker used in the identification of BAT. MPR-11A, BMPR-1B, BMPR-2, and ACVR-1 are adipogenic markers. Pref-1 is an inhibitor of adipogenesis that can be used to confirm adipogenesis. The primers used in the RT-PCT are shown in Example 2 and below. The sense and antisense markers used to amplify UCP-1 were, respectively:
5'-CTGCCAGGACAGTACCCAAG-3' SEQ ID NO: 18
5'-TCAGCTGTTCAAAGCACACA-3' SEQ ID NO:19

The sense and antisense markers used to amplify PRDM-16 were, respectively:
5'-CAGCACGGTGAAGCCATTC-3' SEQ ID NO:20
5'-GCGTGCATCCGCTTGTG-3' SEQ ID NO:21

The sense and antisense markers used to amplify ATPase were, respectively:
5'-ACCTATCCCAGCCTCGTCTC-3' SEQ ID NO:22
5'-AGGACTTGCCCACTTCTCTTT-3' SEQ ID NO:23

The sense and antisense markers used to amplify Pref-1 were, respectively:
5'-AGTACGAATGCTCCTGCACAC-3' SEQ ID NO:24
5'-CTGGCCCTCATCATCCAC-3' SEQ ID NO:25

The sense and antisense markers used to amplify BMPR-1A were, respectively:
5'-AATGCAAGGATTCACCGAAAGCCC-3' SEQ ID NO:26
5'-ACAGCCATGGAAATGAGCACAACC-3' SEQ ID NO:27

The sense and antisense markers used to amplify BMPR-1B were, respectively:
5'-AGAAGAGCACAGAGGCCCAATTCT-3' SEQ ID NO:28
5'-TGCAAGGTACACAGCAGTGCTAGA-3' SEQ ID NO:29

The sense and antisense markers used to amplify BMPR-2 were, respectively:
5'-AGCAATCGCCCATCGAGACTTGAA-3' SEQ ID NO:30
5'-TTCTGGAGGCATATAGCGCTTGGT-3' SEQ ID NO:31

The sense and antisense markers used to amplify ACVR-1 were, respectively:
5'-TGCTAATGATGATGGCTTTCC-3' SEQ ID NO:32
5'-TTCACAGTGGTCCTCGTTCC-3' SEQ ID NO:33

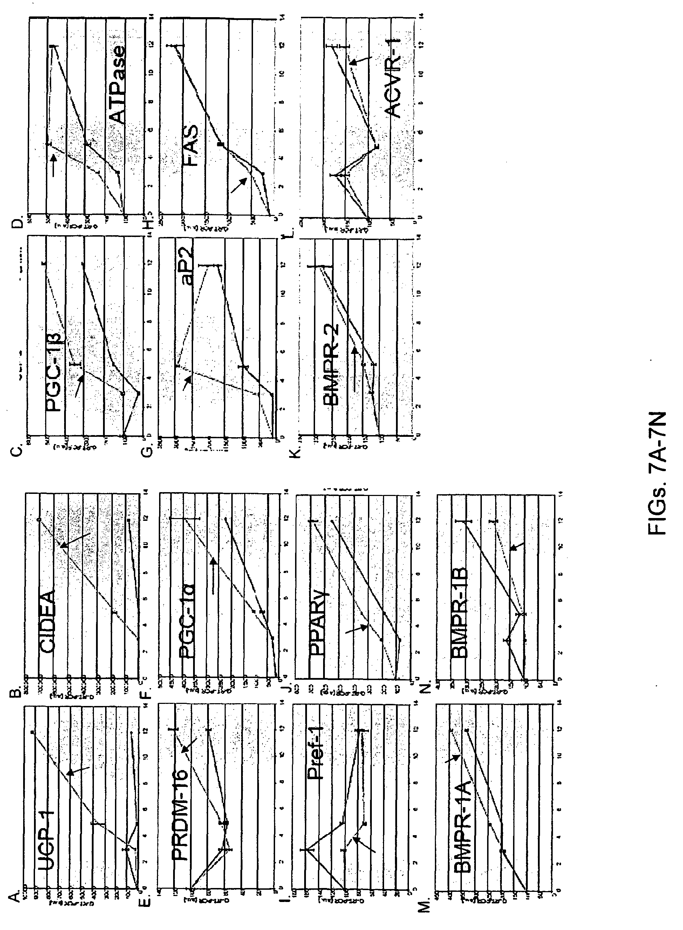

As shown in FIG. 7, BMP7 treatment of Sca-1+ cells resulted in a marked increase in the expression levels of the BAT specific marker UCP-1. Furthermore, UCP-1 expression in these cells continued to increase throughout the 12 days period used in these experiments. An increase in the levels of the BAT specific markers PRDM-16, CIDEA, PGC-1I, and PGC-1ϑ were also observed, as was an increase in the expression levels of ATPase. Increases in the expression levels of the adipogenesis markers, PPARK, aP2, FAS, BMPR-1A, and BMPR-2 were also observed. In contrast, a decrease in the expression level of the adipogenesis inhibitor Pref-1 was observed.

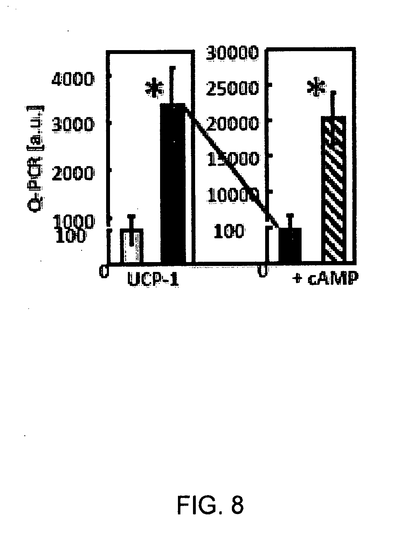

Together, these observations confirm that Sca-1+ cells exposed to BMP7 differentiate not only into adipocytes, but specifically into brown adipocytes. Cells were also exposed to cAMP as described in Example 2, and, as shown in FIG. 8, expression of UCP-1 in these cells was further markedly increased. This observation again supports that Sca-1+ cells become bona fide brown adipocytes with a complete capacity to respond to catecholamine stimulation by activating the thermogenic program.

### Adipose Tissue Derived Sca-1+ Cells

Sca-1+ cells were isolated from adipose tissue, including interscapular BAT (BAT), subcutaneous white adipose tissue (SQ-WAT), and visceral white adipose tissue (EPI-WAT), and cultured using the method described above for skeletal muscle derived Sca-1+ cells. Isolated cells were then exposed to BMP7. The cells abilities to differentiate into BAT was determined using Oil-Red-O staining and RT-PCR.

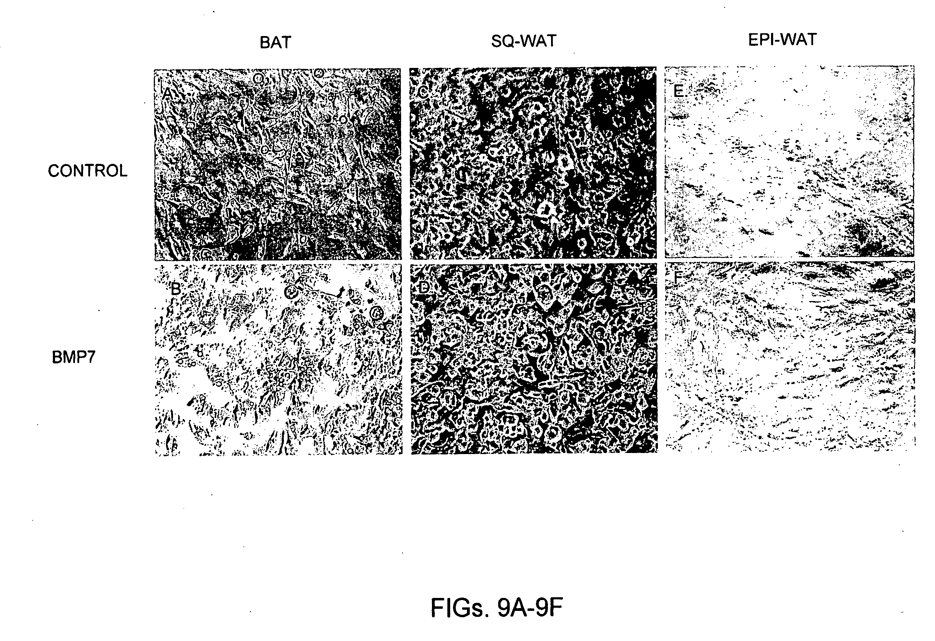

As shown in FIG. 9, Sca-1+ cells isolated from BAT and SQ-WAT adipose tissue responded to BMP7 treatment by differentiating into BAT. In contrast, Sca-1+ cells isolated from EPI-WAT did not.

These visual observations were confirmed using RT-PCR. Sca-1+ were also isolated from BAT (BAT), subcutaneous white adipose tissue (SQ-WAT), and visceral white adipose tissue (EPI-WAT) sources from mice genetically predisposed to obesity, C57B1/6 (B6), and obesity resistant mice, 129-S6 (129). Cells isolated from these mice were then exposed to BMP7 as described above for the muscle derived cells and the markers of the adipogenesis marker FAS and the BAT specific marker UCP-1 were assessed using quantitative RT-PCR.

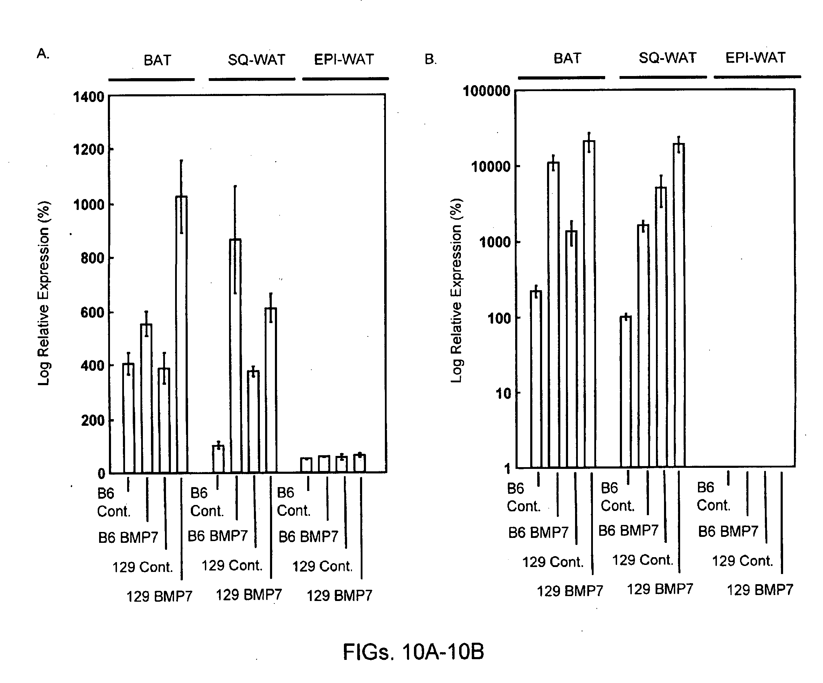

As shown in FIGs. 10A and 10B, the levels of both FAS and UCP-1 increased in Sca-1+ cells isolated from BAT and SQ-WAT obtained from both obesity prone and obesity resistant animals. In contrast, no increase in the level of FAS was observed in Sca-1+ cells obtained from EPI-WAT in either animal and UCP-1 was undetectable. Notably, UCP-1 was expressed at higher levels in Sca-1+ cells isolated from BAT or SQ-WAT of the obesity-resistant 129 mice, and BMP-7 pretreatment further induced UCP-1 expression in these cells.

These observations support that Sca-1+ BAT progenitor cells can be isolated from adipose tissue, and BMP-7 can induce expression of BAT markers.

### Example 4: Muscle resident Sca-1+ Cell Availability and Function In Obesity Prone versus Obesity Resistant Animal Models

The number of Sca-1+ cells in obesity prone C57B1/6 (B6) and obesity resistant 129-S6 (129) mice was assessed using FACS and the cell isolation methods described in Example 3.

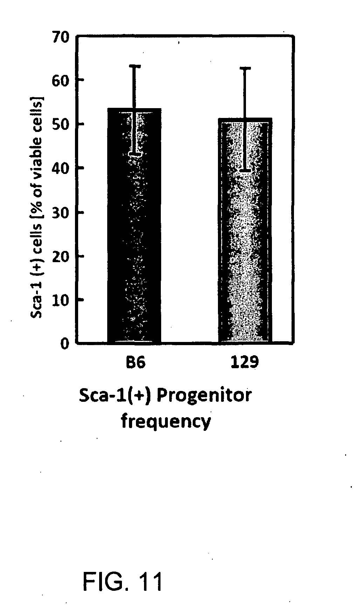

As shown in FIG. 11, the total number of Sca-1+ cells in B6 and 129 is approximately equal. This observation suggests that a genetic predisposition or resistance to obesity does not effect Sca-1+ cell number.

In order to investigate if there is any functional difference in the way cells isolated from the B6 and 129 mice respond to BMP7, Sca-1+ cells from each animal were isolated, cultured, and exposed to BMP7 as described in Example 3. Levels of the BAT specific marker UCP-1 and the adipogenesis marker PPARK were assessed using RT-PCR.

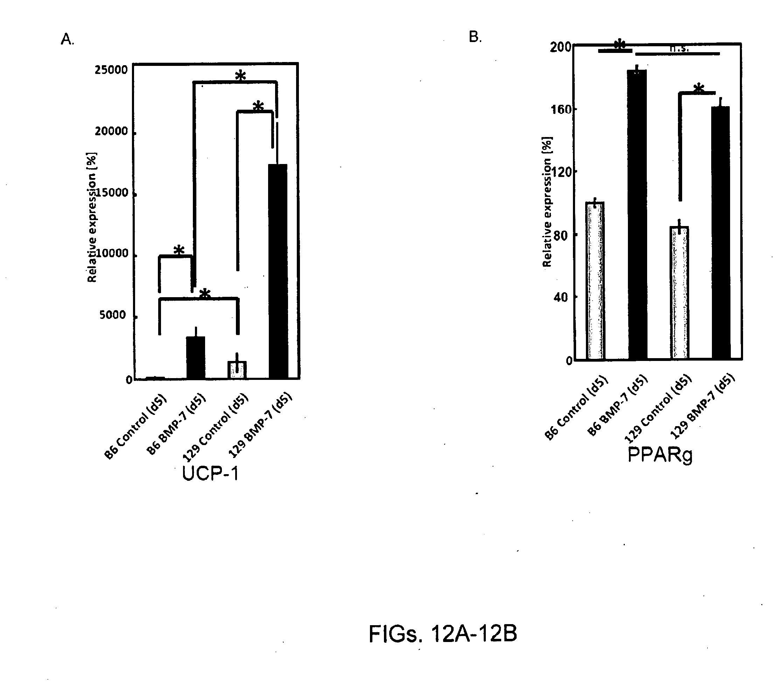

As shown in FIGs. 12A and 12B, BMP7 treatment of Sca-1+ cells isolated from obesity resistant mice (129) resulted in a increase in UCP-1 expression levels that greatly exceeded that observed in obesity prone animals (B6). Baseline levels of UCP-1 were also higher in obesity resistant mice (129) than obesity prone animals (B6). In contrast, PPARK levels were comparable in obesity resistant mice (129) and obesity prone animals (B6).

These observations suggest that genetic background is a determining factor for brown adipogenic potential of Sca-1+ cells.

### Example 5: Ability of BMP7 Treated Sca-1+ Cells to Differentiate into Non-Adipose Cell Lineages

The ability of Sca-1+ cells to differentiate into different lineages with or without BMP pretreatment and in the presence of osteogenic and myogenic, differentiation media was investigated. Specifically, Sca-1+ cells isolated from muscle were pretreated with BMP-7 before being induced to undergo differentiation using osteogenic or myogenic induction cocktail containing hormone and chemicals (Peister et al., Blood 103(5):1662-8 2003). Sca-1+ cells isolated from skeletal muscle that were exposed to BMP7 did not undergo differentiation towards osteogenic or myogenic lineages. These observations suggest that BMP7 specifically predisposes Sca-1+ cells to differentiate to BAT cells. An additional conclusion that can be drawn from these data is that Sca-1+ are adipogenic cells that can differentiate into WAT or BAT, but that exposure of these cells with BMP7 directs the cells to a functional BAT lineage.

### Example 6: In Vivo Implantation of BMP7 Treated Sca-1+ Progenitor Cells

To determine the commitment of these BMP-treated Sca-1+ progenitor cells toward brown versus white adipocyte differentiation *in vivo*, BMP7 treated Sca-1+ cells isolated from genetically engineered green fluorescent protein (GFP) mice were injected into the hindlimbs and/or sternal region of mice of the same genetic background but that do not express GFP.

Using this approach the fate of transplanted cells will be readily followed to determine if the cells possess self-renewal and differentiation capacity *in vivo.*

Cells were isolated from GFP expressing mice and cultured using the methods described in Example 3. These cells were then implanted, as follows. Approximately 5 x 10⁵ cells were injected directly into hindlimb muscle and subcutaneous white fat around the flank region of non-GFP recipient C57BL/6 mice. Ten days after implantation, the BMP-7-treated Sca-1⁺ cells developed into GFP⁺ fat pads in both injection sites as measured by epifluorescence.

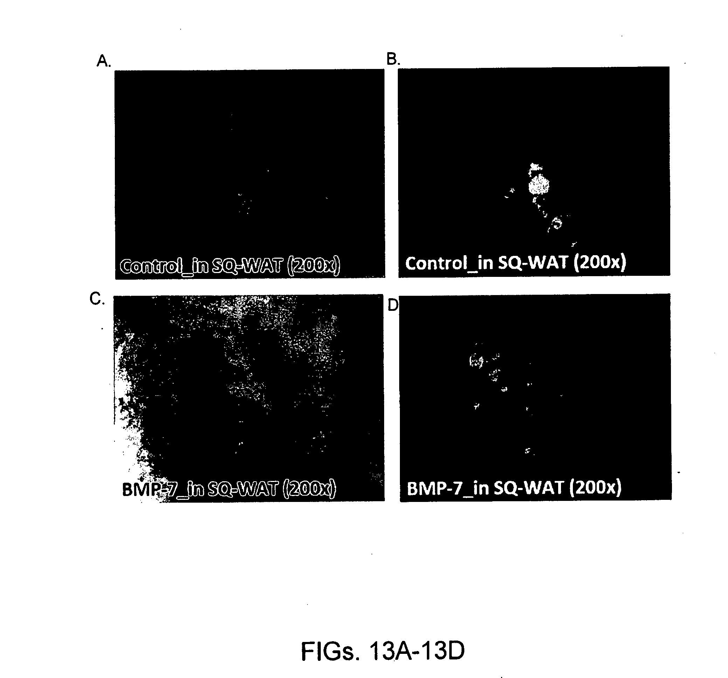

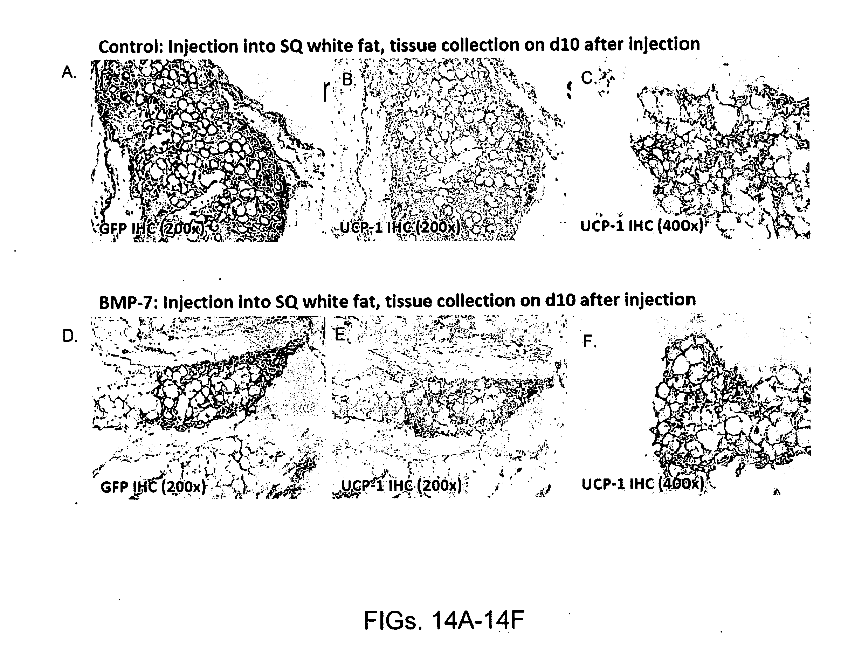

As shown in FIG. 13 and FIG. 14, implanted BMP7 treated Sca-1+ cells were detected at 10 days post implantation in SQ-WAT tissue. Furthermore, BMP7 treated Sca-1+ cells were still detectable 30 days post implantation.

These data suggest that BMP7 treated Sca-1+ cells survive cell implantation and remain viable following implantation.

### Example 7: Treatment of Sca-1+ Cells with BMP3, BMP4, and BMP7

Sca-1+ cells isolated from skeletal muscle using the methods described in Example 3 were exposed to either BMP3, BMP4, or BMP7. The levels of UCP-1, CIDEA, PPARK, FAS, Myogenin, and MyoD were then assessed using quantitative RT-PCR and the methods described in Examples 2 and 3.

The sense and antisense markers used to amplify Myogenin were, respectively:
5'-CTACAGGCCTTGCTCAGCTC-3' SEQ ID NO:34
5'-TGTGGGAGTTGCATTCACTG-3' SEQ ID NO:35

The sense and antisense markers used to amplify MyoD were, respectively:
5'-TGGTTCTTCACGCCCAAAAG-3' SEQ ID NO:36
5'-TCTGGAAGAACGGCTTCGAA-3' SEQ ID NO:37

The sense and antisense markers used to amplify ACVR-1 were, respectively:
5'-TGCTAATGATGATGGCTTTCC-3' SEQ ID NO:38
5'-TTCACAGTGGTCCTCGTTCC-3' SEQ ID NO:39

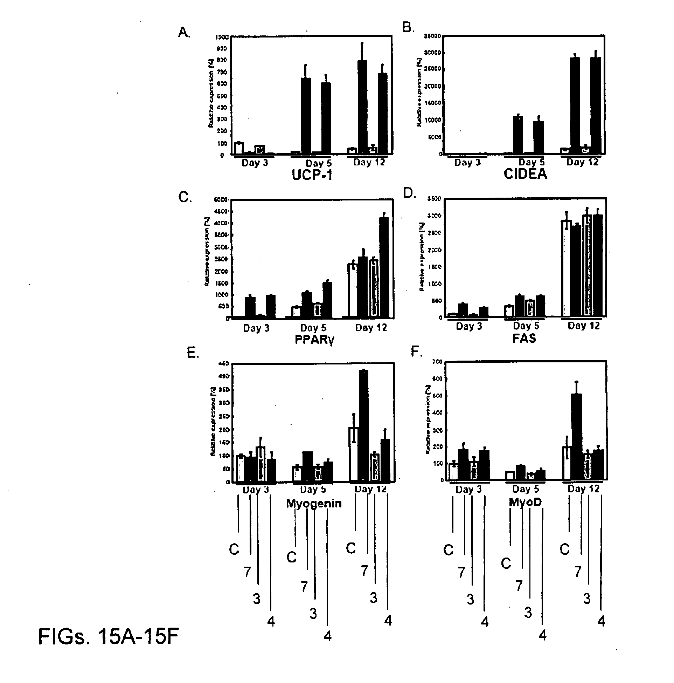

As shown in FIG. 15, BMP7 and BMP4 promote an increase in the expression of the BAT specific markers UCP-1 and CIDEA in Sca-1+ cells.

These data suggest that BMP4 can be used, in addition to BMP7, to promote the differentiation of Sca-1+ progenitor cells to BAT.

### Example 8: Cell Implantation

Sca-1+ cells will also be embedded in a scaffold, matrix or other structure to which the cells can attach to facilitate the implantation. The resulting tissue will be analyzed by general histology and immunohistochemistry of brown- and white fat-specific markers described above. Alterations in body weight, glucose and lipid metabolism, and whole-body energy balance associated with implantation will be closely monitored.

Sca-1+ progenitor cells will also be isolated from other tissues including skeletal muscle, prostate, dermis, the cardiovascular system, mammary gland, liver, neonatal skin, calvaria to determine if the ability to differentiate to generate brown adipocytes is tissue specific.

### Example 9: Characterizing Committed Preadipocytes

Different BMP trigger the commitment of multipotent progenitor cells into brown or white adipocytes. Specifically, BMP-2 and BMP-4 promote differentiation to the white adipocyte lineage, whereas BMP-7 promotes differentiation to the brown adipocyte lineage.

Sca-1+ progenitor cells exposed to BMPs will be characterized using comprehensive genomic and proteomic approaches to determine the molecular signatures and cell surface markers to generate cell profiles that distinguish between committed brown preadipocytes, committed white preadipocytes, and uncommitted stem cells. These profile will be used to identify and select cell populations based on their ability to generate brown or white adipocytes,

The genomics approaches will include the use of Affymetric microarrays for expression profiling and stable isotope labeling in cell culture (SILAC) for proteomics analysis. MicroRNAs will also be studied in BMP treated Sca-1+ progenitor cells.

Thus, cell profile useful for the identification of progenitor cell populations capable of differentiating to brown or white adipocyte lineages will be generated by studying the DNA, RNA, protein, and MicroRNA profiles of Sca-1+ progenitor cells exposed to BMPs.

### Example 10: Analysis of the Physiological Relevance of Brown and White Progenitors in the Treatment of Diabetes

Sca-1+ progenitor cells and the mature brown adipocytes resulting from BMP-7 treatment of these cells are transplanted into obese mice. Animals are then studied to assess changes in body weight, metabolism, and or diabetes.

Cells identified using the methods described herein are injected into experimental animal models with distinct susceptibilities to develop obesity. Suitable animals are the obesity-resistant strain 129S6/SvEvTac, the obesity prone strain C57BL/6, *ob*/*ob* mice, and *db*/*db* mice, the latter two of which are deficient in leptin and leptin receptor, respectively. Weight gain and/or obesity are also assessed using the methods described by Rogers and Webb (Rogers P. and Webb G.P., Br. J. Nutr. 43:83-86, 1980).

Experiments are then performed to evaluate the effects of (1) diets containing different amounts or ratios of fats, carbohydrates, or proteins, (2) treatment with pharmaceutical agents including the anti-diabetic drug rosiglitazone, and/or (3) exercise on control animals (that have not received cells) and experimental animals (that have received transplanted cells).

Control and experimental animals are then assessed before and after transplantation and throughout testing. Animals are tested by analyzing changes in body weight (by weighing a live animal), serum glucose (by measuring serum glucose levels in a live animal), and/or serum insulin (by measuring serum insulin levels). Some animals are then sacrificed and used to further analysis. Other animals are used for long-term studies. Other animals are used for metabolic assessments using the Comprehensive Lab Animal Monitoring System (CLAMS, Columbus Instruments, Columbus, OH), which is capable of measuring spatial activity, food and liquid consumption, temperature, urine production, metabolic rate and oxygen and carbon dioxide exchange.

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

## Claims

1. A method of increasing the number of cells with the characteristics of brown adipose tissue (BAT) cells in a population of cells *in vitro*, the method comprising:
contacting a population of cells comprising stem cell antigen-1 positive (Sca-1+) progenitor cells, wherein the population of progenitor cells comprises at least 60% Sca-1+ progenitor cells, *in* vitro with an effective amount of bone morphogenic protein (BMP)-7 and, optionally, up to three of bone morphogenic protein (BMP) -2, -4, or -6 or BMP-4 and, optionally, up to three of BMP-2, -6 or -7 for a time sufficient to increase the number of cells with the characteristics of brown adipose tissue (BAT) cells in the population of cells; or
genetically engineering the population of cells wherein the population of cells comprises stem cell antigen-1 positive (Sca - 1+) progenitor cells, wherein the population of progenitor cells comprises et least 60% Sca-1+ progenitor cells, to express bone morphogenic protein (BMP)-7 and, optionally, up to three of bone morphogenic protein (BMP) -2, -4, or -6 or BMP-4 and, optionally, up to three of BMP-2, -6 or -7 at a level sufficient to increase the number of cells with the characteristics of BAT cells in the population of cells.

2. The method of Claim 1, further comprising:
culturing the population of progenitor cells in a first cell growth medium comprising 0.1 - 100 mM insulin and 0.1 - 10 nM triiodothyronie (T3), 0.1 - 5 µM isobutylmethylxanthine (IBMX), 0.1 - 50 mM dexamethasone, and 0.01 - 10 mM indomethacin; and culturing the population of progenitor cells in a second cell growth medium comprising insulin and T3.

3. The method of Claims 1 and 2, further comprising contacting the population of progenitor cells with one or more of BMP-1, BMP-3, peroxisone proliferator-activated receptor gamma (PPARγ), Retinoid X receptor alpha (RxRα), T3, a thiazolidimedione (TZD), vitamin A, retinoic acid, glucocrticoid or agonist thereof, Wingless-type (Wnt), Insulin-like growth factor Factor-1 (IGF-1), Epidermal growth factor (EGF), Fibroblast growth factor (FGF), Transforming growth factor (TGF)-α, TGF-β, Tumor necrosis factor alpha (TNFα), Macrophage colony stimulating factor (MCSF), Vascular endothelial growth factor (VEGF) and Platelet-derived growth factor (PDGF).

4. The method of Claim 1, wherein the population of progenitor cells is obtained from skeletal muscle, prostate tissue, dermis tissue, tissue from the cardiovascular system, mammary glade tissue, liver tissue, neonatal skin, calvaria, bone marrow, intestinal tissue, adipose tissue, and adipose tissue deposits.

5. The method of Claim 1, wherein the population of progenitor cells is obtained from skeletal muscle.

6. The method of Claim 1, wherein the population of progenitor cells comprises at least 70% Sca-1+ progenitor cells.

7. The method of Claim 1, wherein the method additionally comprises contacting the population of progenitor cells with a BMP-2, BMP-4, BMP-6, BMP-7 agonist.

8. The method of Claim 1, wherein the method additionally comprises contacting the population of progenitor cells with a BMP-7 or BMP-4 agonist.

9. The method of Claim 1, wherein the cells with characteristics of BAT cells express higher levels of uncoupling protein (UPC-1) and cell death-inducing DFF45-like effector A (CIDEA) compared to untreated Sca-1+ progenitor cells.

10. The method of Claim 2, wherein the first cell growth medium comprises 20 nM insulin and 1 nM triiodothyronine (T3), 0.5 µM isobutylmethylxanthine (IBMX), 5 mM dexamethasone, and 0.125 mM idomethacin.

## Patentansprüche

1. Verfahren zum Erhöhen der Anzahl von Zellen mit den Eigenschaften von braunen Fettgewebezellen (BAT-Zellen) in einer Zellpopulation in vitro, wobei das Verfahren aufweist:
Inkontaktbringen einer Population von Zellen, die Stammzellen-Antigen-1-positive (Sca-1+) Vorläuferzellen aufweist, wobei die Population von Vorläuferzellen mindestens 60% Sca-1+-Vorläuferzellen aufweist, in vitro mit einer wirksamen Menge knochenmorphogenem Protein (BMP)-7, und wahlweise bis zu drei knochenmorphogenen Proteinen (BMP)-2, -4 oder -6, oder BMP-4 und wahlweise bis zu drei BMP-2, -6 oder -7, während einer Zeit, die ausreicht, um die Anzahl von Zellen mit den Eigenschaften von braunen Fettgewebezellen (BAT-Zellen) in der Zellpopulation zu erhöhen; oder
Genmanipulation der Zellpopulation, wobei die Zellpopulation Stammzellen-Antigen-1-positive (Sca-1+-) Vorläuferzellen aufweist, wobei die Population von Vorläuferzellen mindestens 60% Sca-1+-Vorläuferzellen aufweist, um knochenmorphogenes Protein (BMP)-7 und wahlweise bis zu drei knochenmorphogene Proteine (BMP)-2, -4 oder -6, oder BMP-4 und wahlweise bis zu drei BMP-2, -6 oder -7 in einer Konzentration zu exprimieren, die ausreicht, um die Anzahl von Zellen mit den Eigenschaften von BAT-Zellen in der Zellpopulation zu erhöhen.

2. Verfahren nach Anspruch 1, das ferner aufweist:
Kultivieren der Population von Vorläuferzellen in einem ersten Zellwachstumsmedium, das 0,1-100 mM Insulin und 0,1-10 nM Triiodthyronin (T3), 0,1-5 µM Isobutylmethylxanthin (IBMX), 0,1-50 mM Dexamethason und 0,01-10 mM Indomethacin aufweist; und Kultivieren der Population von Vorläuferzellen in einem zweiten Zellwachstumsmedium, das Insulin und T3 aufweist.

3. Verfahren nach Anspruch 1 und 2, das ferner das Inkontaktbringen der Population von Vorläuferzellen mit einer oder mehreren der folgenden Verbindungen aufweist: BMP-1, BMP-3, Peroxisom-Proliferator-aktiviertem Rezeptor gamma (PPARγ), Retinoid-X-Rezeptor alpha (RxRα), T3, einem Thiazolidindion (TZD), Vitamin A, Retinsäure, Glucocorticoid oder einem Agonisten davon, Wingless-Type (Wnt), insulinähnlichem Wachstumsfaktor Faktor-1 (IGF-1), epidermalem Wachstumsfaktor (EGF), Fibroblastenwachstumsfaktor (FGF), transformierendem Wachstumsfaktor (TGF)-α, TGF-β, Tumornekrosefaktor alpha (TNF-α), Makrophagenkoloniestimulierendem Faktor (MCSF), Gefäßendothelwachstumsfaktor (VEGF) und aus Blutplättchen stammendem Wachstumsfaktor (PDGF).

4. Verfahren nach Anspruch 1, wobei die Population von Vorläuferzellen aus Skelettmuskel, Prostatagewebe, Dermisgewebe, Gewebe aus dem kardiovaskulären System, Brustdrüsengewebe, Lebergewebe, neonataler Haut, Calvaria, Knochenmark, Darmgewebe, Fettgewebe und Fettgewebeablagerungen gewonnen wird.

5. Verfahren nach Anspruch 1, wobei die Population von Vorläuferzellen aus Skelettmuskel gewonnen wird.

6. Verfahren nach Anspruch 1, wobei die Population von Vorläuferzellen mindestens 70% Sca-1+-Vorläuferzellen aufweist.

7. Verfahren nach Anspruch 1, wobei das Verfahren zusätzlich das Inkontaktbringen der Population von Vorläuferzellen mit einem BMP-2-, BMP-4-, BMP-6- oder BMP-7-Agonisten beinhaltet.

8. Verfahren nach Anspruch 1, wobei das Verfahren zusätzlich das Inkontaktbringen der Population von Vorläuferzellen mit einem BMP-7- oder BMP-4-Agonisten beinhaltet.

9. Verfahren nach Anspruch 1, wobei die Zellen mit Eigenschaften von BAT-Zellen im Vergleich zu unbehandelten Sca-1+-Vorläuferzellen höhere Konzentrationen von Entkopplungsprotein (UCP-1) und Zelltod-induzierendem DFF45-ähnlichem Effektor A (CIDEA) exprimieren.

10. Verfahren nach Anspruch 2, wobei das erste Zellwachstumsmedium 20 nM Insulin und 1 nM Triiodthyronin (T3), 0,5 µM Isobutylmethylxanthin (IBMX), 5 mM Dexamethason und 0,125 mM Indomethacin aufweist.

## Revendications

1. Procédé destiné à augmenter le nombre de cellules ayant les caractéristiques des cellules du tissu adipeux brun (BAT), dans une population de cellules *in vitro,* le procédé comprenant:
mettre une population de cellules comprenant des cellules progénitrices de cellules souches antigène 1-positives (Sca-1+), où la population de cellules progénitrices comprend au moins 60 % de cellules progénitrices Sca-1+, en contact *in vitro* avec une quantité efficace de protéine morphogénique de l'os (BMP)-7 et, optionnellement, jusqu'à trois des protéines morphogéniques de l'os (BMP)-2, -4 ou -6 ou BMP-4 et, optionnellement, jusqu'à trois de BMP-2, -6 ou -7 pendant un temps suffisant pour augmenter le nombre de cellules ayant les caractéristiques des cellules du tissu adipeux brun (BAT) dans la population de cellules; ou bien
manipuler génétiquement la population de cellules, où la population de cellules comprend des cellules progénitrices de cellules souches antigène 1-positives (Sca-1+), où la population de cellules progénitrices comprend au moins 60 % de cellules progénitrices Sca-1+, pour exprimer la protéine morphogénique de l'os (BMP)-7 et, optionnellement, jusqu'à trois des protéines morphogéniques de l'os (BMP)-2, -4 ou -6 ou BMP-4 et, optionnellement, jusqu'à trois de BMP-2, -6 ou -7 à un niveau suffisant pour augmenter le nombre de cellules ayant les caractéristiques des cellules BAT dans la population de cellules.

2. Procédé selon la revendication 1, comprenant en outre:
cultiver la population de cellules progénitrices dans un premier milieu de croissance cellulaire comprenant de l'insuline 0,1-100 mM et de la triiodothyronine 0,1-10 nM (T3), de l'isobutylméthylxanthine 0,1-5 µM (IBMX), de la dexaméthasone 0,1-50 mM et de l'indométhacine 0,01-10 mM; et cultiver la population de cellules progénitrices dans un deuxième milieu de croissance cellulaire comprenant de l'insuline et de la T3.

3. Procédé selon les revendications 1 et 2, comprenant en outre mettre la population de cellules progénitrices en contact avec un ou plusieurs d'entre BMP-1, BMP-3, récepteur gamma activé par les proliférateurs de peroxysomes (PPARγ), récepteur des rétinoïdes X type alpha (RxRα), T3, une thiazolidinedione (TZD), vitamine A, acide rétinoïque, glucocorticoïde ou agoniste de celui-ci, type Wingless (Wnt), facteur 1 de croissance insulinique (IGF-1), facteur de croissance épidermique (EGF), facteur de croissance des fibroblastes (FGF), facteur de croissance de transformation (TGF)-α, TGF-β, facteur alpha de nécrose tumorale (TNFα), facteur de stimulation des colonies de macrophages (MCSF), facteur de croissance de l'endothélium vasculaire (VEGF) et facteur de croissance dérivé des plaquettes (PDGF).

4. Procédé selon la revendication 1, dans lequel la population de cellules progénitrices est obtenue de muscle squelettique, tissu de la prostate, tissu dermique, tissu du système cardiovasculaire, tissu de la glande mammaire, tissu hépatique, peau néonatale, sinciput, moelle osseuse, tissu intestinal, tissu adipeux et dépôts de tissus adipeux.

5. Procédé selon la revendication 1, dans lequel la population de cellules progénitrices est obtenue de muscle squelettique.

6. Procédé selon la revendication 1, dans lequel la population de cellules progénitrices comprend au moins 70 % de cellules progénitrices Sca-1+.

7. Procédé selon la revendication 1, où le procédé comprend en plus mettre la population de cellules progénitrices en contact avec un agoniste de BMP-2, BMP-4, BMP-6, BMP-7.

8. Procédé selon la revendication 1, où le procédé comprend en plus mettre la population de cellules progénitrices en contact avec un agoniste de BMP-7 ou de BMP-4.

9. Procédé selon la revendication 1, dans lequel les cellules ayant les caractéristiques des cellules BAT expriment des niveaux élevés de protéine de découplage (UPC-1) et de l'effecteur A inducteur de mort cellulaire analogue à DFF45 (CIDEA), comparées aux cellules progénitrices Sca-1+ non traitées.

10. Procédé selon la revendication 2, dans lequel le premier milieu de croissance cellulaire comprend de l'insuline 20 nM et de la triiodothyronine 1 nM (T3), de l'isobutylméthylxanthine 0,5 µM (IBMX), de la dexaméthasone 5 mM et de l'indométhacine 0,125 mM.
